(19) Europäisches Patentamt
European Patent Office
Office européen des brevets

(11) **EP 4 740 933 A1**

(12) **EUROPEAN PATENT APPLICATION**
published in accordance with Art. 153(4) EPC

(43) Date of publication:
**13.05.2026 Bulletin 2026/20**

(21) Application number: **24838633.6**

(22) Date of filing: **01.07.2024**

(51) International Patent Classification (IPC):
**A61K 8/44** (2006.01)    **A61K 8/64** (2006.01)
**A61Q 19/10** (2006.01)   **A61Q 5/02** (2006.01)
**A61Q 19/00** (2006.01)   **C11D 7/34** (2006.01)
**C11D 7/32** (2006.01)

(52) Cooperative Patent Classification (CPC):
**A61K 8/44; A61K 8/64; A61Q 5/02; A61Q 19/00;
A61Q 19/10; C11D 7/32; C11D 7/34**

(86) International application number:
**PCT/CN2024/102882**

(87) International publication number:
**WO 2025/011378 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.07.2023   CN 202310833176
03.11.2023   CN 202311455989
03.11.2023   CN 202311455982
03.11.2023   CN 202311456029**

(71) Applicant: **Suzhou Oulit Biopharm Co., Ltd.
Suzhou, Jiangsu 215411 (CN)**

(72) Inventor: **ZHANG, Jian
Suzhou, Jiangsu 215122 (CN)**

(74) Representative: **Winger
Mouterij 16 bus 101
3190 Boortmeerbeek (BE)**

(54) **CLEANING COMPOSITION AND USE THEREOF**

(57)   A cleaning composition, related cleaning products, and a use of the composition in reduction of skin or eye irritation, oil control, deodorization and other aspects. The present invention takes N-long-chain acyl amino acid and alkaline amino acid/inorganic base as main surfactants without containing a thickening agent, ethoxylate, a fatty acid salt, and a moisturizer, is mild and non-irritating, and can be used for bath cream, shampoo, facial cleansing mousse, a makeup remover, an oral cavity washing product, a shaving product, a hand sanitizer, a cleaning lotion or a multifunctional cleaning product.

FIG. 4

## Description

### TECHNICAL FIELD

The present disclosure relates to the field of cleaning composition technology, and in particular, to a cleaning composition comprising a N-long-chain acyl amino acid and a basic amino acid/inorganic base as the primary surfactant, and use thereof in cosmetics and various cleaning products.

### BACKGROUND

In the past two decades, washing products have been more and more subdivided in the aspects of categories, concepts, effects, channels, consumer populations, etc. However, the ultimate subdivision also brings troubles to many consumers in selection and use, such that the concept of "multi-effect and all-in-one" of the product is increasingly mentioned.

At present, the facial cleansing, makeup removal, shaving, body wash, and shampoo are completely different product types, and have different formula design concepts, ingredient combinations, and the like.

Facial cleansing products mainly consist of an alcohol (for dissolving, dispersing, and other effects), a surfactant such as sodium lauroyl glutamate, a co-surfactant (for foaming and other effects), a filler such as modified starch (for adjusting the paste-forming point, filling and softening paste), an emulsifier such as A165 (emulsifying part of unneutralized fatty acids or part of alcohol, lipids, etc.), a humectant, a preservative, a fragrance, etc.

Body wash products mainly consist of a primary surfactant (generally, sodium laureth sulfate) + a co-surfactant (cocamidopropyl betaine, etc.) + a thickening agent + an ester supplement (jojoba oil, etc.) + a conditioner (a polyol) + an adjunct.

Shampoo products mainly are composed of: water + a surfactant (the primary surfactant is generally sodium laureth sulfate) + a thickening agent + a pH regulator + a cationic conditioner + a fragrance + a chelating agent + a preservative, etc., and the auxiliary (optionally added) ingredients include: a viscosity regulator, an anti-dandruff agent, a pearlizing agent, a humectant, a pigment, a plant extract, etc.

As representative makeup removal products, the makeup removal oil mainly consists of a light lipid + a surfactant + an active ingredient, while the makeup removal lotion mainly consists of water + a light lipid + a surfactant + an active ingredient. The makeup removal water mainly consists of water + a surfactant + an active ingredient. The surfactant is generally a polyglycerol ester surfactant. Common surfactants in the makeup removal water include: PEG-6 caprylic/-capric glyceride, PEG-7 caprylic/capric glyceride, PEG-8 caprylic/capric glyceride, PEG-7 glyceryl cocoate, poloxamer 184, Tween series, and the like.

For shaving products, the formulas are generally subjected to conventional soap-based emulsification, resulting in a dispersion system of alkali metal soap in glycerol and water. Fatty acid salts are used as the emulsifier to produce abundant and fine foams. In order to improve the comfort of the skin, some additional emollients, humectants, cooling agents, and astringents may be added to the formula.

Conventional anionic surfactants such as alkyl sulfates and polyoxyethylene alkyl sulfates have been widely used as the surfactant for hair/body cleaning bases. However, with the increasing use of shampoo/body wash products in recent years, they may sometimes lead to dry and irritated feels. There is a need for cleansers with less irritation. In addition, such surfactants are petroleum-derived surfactants, contrary to the current sustainable development and environmental conservation orientation.

I. Sulfate surfactants

Most current cleaning products are based on surfactants containing sulfates, such as sodium lauryl sulfate (SLS), ammonium lauryl sulfate (ALS), sodium laureth sulfate (SLES), or ammonium laureth sulfate (ALES). These sulfate surfactants integrate the advantages of cost-efficiency, satisfying cleaning and foaming performance, etc. However, as described in EP3582748A1, it is known that surfactants containing sulfate salts (e.g., SLS) tend to cause tolerance problems, especially on the skin and eyes. Another disadvantage of sulfate-containing surfactants is their tendency to remove natural lipids, fats or proteins on the surface of the skin, scalp, or hairs. In the long term, repeated use of personal cleaning products comprising sulfate-containing surfactants may cause irritation to the skin or scalp and/or result in damage to hairs.

II. Ethoxylate surfactants

The most typical ethoxylate surfactant is sodium laureth sulfate (SLES or AES). AES is widely used due to its good detergency and ease to thicken by adding sodium chloride (although it is convenient to add a large amount of sodium chloride for thickening, the irritation of sodium chloride to the eyes is usually underestimated). Although milder than alkyl sulfates, it is still moderately irritant (see GB2598154A). In addition, ethoxylate surfactants contain a large amount of non-renewable carbon from ethylene oxide and thereby cause safety concerns (dioxane contained in the surfactant is carcinogenic).

III. Fatty acid salts

Fatty acid salts have been used as an essential component of body cleaning compositions such as body washes, hand soaps, and facial cleansers due to their good foaming and refreshing feels. Fatty acid salts have good cleaning and foaming performance, but can only be used to prepare products with high pH (> 9.0). They may dry and irritate the skin, resulting in undesirable skin feels (see GB2598154A and EP3957297A1).

Similarly, CN105997546B discloses a cleaning composition using fatty acid salts as the active ingredient, which also has the specific problems of tension and dryness feels caused by fatty acid salts after drying. Moreover, in body wash compositions, the state of the washed skin is important in addition to the detergency or foam. As a characteristic of fatty acid salts, tightness on the skin can be felt after use. Furthermore, the use of water of a high hardness may lead to a stronger tightness feel. The tightness feel is generally undesirable.

IV. Amino acid surfactants

The cosmetic and personal care industries have confirmed the increasing popularity and importance of "sulfate-free", "dioxane-free" personal care cleaning products containing environmentally-friendly, sustainable, and mild surfactants. Amino acid-based surfactants are considered to be "greener", milder, and more sustainable than conventional petroleum-based surfactants.

The development of high-quality and environment-friendly surfactants has become a main orientation of the surfactant industry, and to replace conventional petroleum-based surfactants with amino acid surfactants is the progress trend of the industry. Unilever has announced the halving of petroleum-based product use in 2025 and phaseout by 2030. L'Oréal has announced a 95% replacement of petroleum-based products by 2030. Therefore, it is necessary to explore the formulation system of amino acid surfactants as the primary surfactant. Most of the current studies focus on the combination of amino acid surfactants with conventional petroleum-based surfactants or the thickening of amino acid surfactants, and there are few studies on how to reduce skin irritation or eye irritation, improve the ease-to-rinse performance, control oil, and the like. In addition, if the acyl amino acid anionic surfactant is formulated as the active cleaning ingredient according to conventional formulas, consumers may easily feel sticky (or squishy), which, in general, is also an undesirable feel.

No-rinse or dry-cleanable shampoo products are especially important for some special populations, such as the elderly, patients, infants, women in postpartum period, those on travelling or business trip, border guards, and office workers who want quick hair wash or cleaning. Hair treatments with an aerosol shampoo containing no surfactants but a solvent and a propellant are known in the art. By spraying the shampoo onto the hair using an aerosol device, when the solvent has evaporated, the starch granules contained in the dry shampoo absorb the sebum and dirt and are brushed off after a preset reaction time. However, hair treated with the aerosol shampoo may be less silky and fluffy, and may even be sticky. Powders and debris also pose difficulties in removal and may leave white/off-white residues. In addition, the use of propellants should be avoided for environmental conservation and safety reasons.

At present, there is a growing number of families keeping cats and dogs. Their thick and long hair is prone to having dirt, carrying various pathogenic microorganisms, leading to skin diseases. Cleaning products for pets on the market may easily cause a certain irritation to pets, and cannot meet the requirements of dry-cleanable or no-rinse cleaners, which may result in excessive water use, frequent and prolonged washes, and pet noncompliance.

There is a desire for hair cleansers that not only have good detergency but also have reduced irritation to eyes and scalp and an excellent feel of use.

## SUMMARY

In view of this, in one aspect, the present disclosure is intended to provide a cleaning composition, comprising a N-long-chain acyl amino acid and a basic amino acid/inorganic base as the primary surfactant, where the basic amino acid is particularly preferred. The composition combines advantages of low irritation, selective cleaning/detergency, ease to rinse, no stickiness, and the like.

In another aspect, the present disclosure provides a no-rinse cleaning product or a dry-cleanable cleaning product.

In yet another aspect, the present disclosure is intended to provide a method for reducing the skin irritation or eye irritation in a cleaning composition.

In still another aspect, the present disclosure is intended to provide a method for improving the easy-to-rinse or water-saving performance in a cleaning composition.

In still another aspect, the present disclosure provides a method for improving the deodorization performance in a cleaning composition.

In the last aspect, the present disclosure is intended to provide a method for imparting the oil control performance to a cleaning composition.

Specifically, the present disclosure discloses the following schemes.

A cleaning composition, comprising: (a) a primary surfactant, (b) a co-surfactant, and (c) water, wherein the percentage by weight of the primary surfactant in all surfactants is greater than 50 wt%, preferably greater than 65 wt%, and more preferably greater than 80 wt%.

The primary surfactant consists of a N-long-chain acyl amino acid and a basic amino acid; preferably, the percentage by weight of the N-long-chain acyl amino acid in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;

or, the primary surfactant consists of a N-long-chain acyl amino acid, a N-long-chain acyl amino acid dipeptide, and a basic amino acid; preferably, the sum of the percentages by weight of the N-long-chain acyl amino acid and the N-long-chain acyl amino acid dipeptide in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;

or, the primary surfactant consists of a N-long-chain acyl amino acid and an inorganic base; preferably, the percentage by weight of the N-long-chain acyl amino acid in the cleaning composition is 1-12 wt%, preferably 2-10 wt%, and more preferably 4-8 wt%, and the percentage by weight of the inorganic base in the cleaning composition is 0.1-3 wt%, preferably 0.2-2.5 wt%, and more preferably 0.4-2 wt%;

the percentage by weight of the co-surfactant in the cleaning composition is 0-10 wt%, preferably 0-5 wt%, and more preferably 0-1 wt%.

The cleaning composition according to [1], wherein the cleaning composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a polyol humectant, preferably completely free of a polyol humectant.

The cleaning composition according to [1] or [2], wherein the co-surfactant comprises one or more of a nonionic surfactant, a cationic surfactant, an amphoteric surfactant, and a sodium salt, a potassium salt and a TEA salt of a N-long-chain acyl amino acid.

The cleaning composition according to any one of [1]-[3], wherein the co-surfactant is substantially free of a nonionic surfactant, preferably, completely free of a nonionic surfactant.

The cleaning composition according to any one of [1]-[4], wherein the co-surfactant is substantially free of a cationic surfactant, preferably, completely free of a cationic surfactant.

The cleaning composition according to any one of [1]-[5], wherein the co-surfactant is substantially free of an amphoteric surfactant, preferably, completely free of an amphoteric surfactant.

The cleaning composition according to any one of [1]-[6], wherein the percentage by weight of the co-surfactant in the cleaning composition is 0 wt%, i.e., the percentage by weight of the primary surfactant in all surfactants is 100 wt%.

The cleaning composition according to any one of [1]-[7], wherein the N-long-chain acyl group in the N-long-chain acyl amino acid is derived from a saturated or unsaturated, linear or branched fatty acid with 8-22 carbon atoms; and/or, the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, or (methyl) taurine.

The cleaning composition according to any one of [1]-[8], wherein the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl; and/or, the amino acid in the N-long-chain acyl amino acid is derived from one or more of alanine, glycine, glutamic acid, sarcosine, arginine, lysine, or (methyl) taurine, preferably alanine, and most preferably L-alanine.

The cleaning composition according to any one of [1]-[9], wherein the basic amino acid is selected from one or more of arginine, lysine, citrulline, ornithine, creatine, histidine, diaminobutyric acid, and diaminopropionic acid, preferably arginine and/or lysine, and most preferably arginine;

and/or, the inorganic base is selected from one or more of sodium hydroxide and potassium hydroxide.

The cleaning composition according to any one of [1]-[10], wherein the N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl;

and/or, the amino acids in the N-long-chain acyl amino acid dipeptide are derived from one or more of alanine, glycine, glutamic acid, sarcosine, arginine, lysine, or (methyl) taurine, preferably alanine, and most preferably L-alanine.

The cleaning composition according to any one of [1]-[11], wherein the N-long-chain acyl amino acid dipeptide is selected from one or more of N-long-chain acyl glycyl glycine, N-long-chain acyl glutamyl glutamic acid, N-long-chain acyl alanyl alanine, and N-long-chain acyl sarcosyl sarcosine, preferably N-long-chain acyl alanyl alanine, and most preferably N-lauroyl-L-alanyl-L-alanine.

The cleaning composition according to any one of [1]-[12], wherein the percentage by weight of the N-long-chain acyl amino acid dipeptide in the composition is 0.1 wt% or greater, preferably 0.3 wt% or greater, and more preferably 0.5 wt% or greater;

and/or, the mass ratio of the N-long-chain acyl amino acid to the N-long-chain acyl amino acid dipeptide is 20:1 to 1.5:1, preferably 15:1 to 3:1, and more preferably 10:1 to 6:1.

The cleaning composition according to any one of [1]-[13], wherein the basic amino acid is present in an amount partially neutralizing the N-long-chain acyl amino acid, preferably with the degree of neutralization being 85% or greater and less than 100%, and more preferably 88% or greater and 96% or less;

or, the basic amino acid is present in an amount partially neutralizing the N-long-chain acyl amino acid and the N-long-chain acyl amino acid dipeptide, preferably with the degree of neutralization being 85% or greater and less than 100%, more preferably 88% or greater and 96% or less;

or, the inorganic base is present in an amount partially neutralizing the N-long-chain acyl amino acid, preferably with the degree of neutralization being 85% or greater and less than 100%, more preferably 88% or greater and 96% or less.

The cleaning composition according to any one of [1]-[14], further comprising (d) a preservative, wherein the preservative is benzoic acid.

The cleaning composition according to any one of [1]-[15], further comprising (e) a perfume or a fragrance, wherein preferably the composition is clear after the addition of the perfume or the fragrance.

The cleaning composition according to any one of [1]-[16], further comprising (f) hyaluronic acid or sodium hyaluronate.

The cleaning composition according to any one of [1]-[17], wherein the pH of the composition is 5-7.5, preferably 5.5-6.9, and more preferably 6-6.7;

and/or, the composition possesses a low viscosity; the low viscosity refers to a viscosity at 25 °C of less than 150 mPa·s, preferably less than 120 mPa·s, and more preferably less than 100 mPa·s.

The cleaning composition according to any one of [1]-[18], wherein for the detergency of the composition on a specified stained fabric as measured by the test method in QB/T1224-2012 Liquid Detergent for Fabric, the JB-01 is 0.8 or higher, preferably 0.85 or higher, and more preferably 0.9 or higher; the JB-02 is 0.8 or higher, preferably 0.85 or higher, and more preferably 0.9 or higher; the JB-03 is 0.4 or higher and 0.8 or lower, preferably 0.45 or higher and 0.75 or lower, and more preferably 0.5 or higher and 0.7 or lower.

The cleaning composition according to any one of [1]-[19], wherein the cleaning composition has better foaming performance in hard water than in soft water; and/or, the foam volumes at 30 s, 3 min, and 5 min in hard water are greater than 400 mL, preferably greater than 450 mL, and more preferably greater than 500 mL; standard for the aforementioned test: GB/T7462-1994 "Surfactant: Measurement of Foaming Performance-Modified Ross-Miles Method", instrument: 2152 modified Ross-Miles foaming tester.

The cleaning composition according to any one of [1]-[20], wherein the cleaning composition is a body wash, a shampoo, a facial mousse, a makeup remover, a mouth wash, a shaving product, a hand sanitizer, a cleaning lotion, or a multi-functional cleaning product.

Use of the cleaning composition according to any one of [1]-[21] in a no-rinse cleaning product or a dry-cleanable cleaning product.

A no-rinse cleaning or a dry-cleanable cleaning product, comprising the cleaning composition according to any one of [1]-[21], wherein preferably, the product is in the form of a cleaning foam or a shampoo.

Use of the cleaning composition according to any one of [1]-[21] in treating or cleaning dry, aged, or damaged skin, or in intimate care.

A cleaning product for infants or the elderly, comprising the cleaning composition according to any one of [1]-[21], wherein preferably, the cleaning product for the infants or the elderly is a cleaning foam, a cleaning mousse, or a two-in-one cleanser for shampoo and body wash.

A method for reducing the skin irritation or eye irritation in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;

the cleaning composition may be the cleaning composition according to any one of [1]-[21];
the method comprises: selecting a primary surfactant consisting of a N-long-chain acyl amino acid and a basic amino acid, wherein preferably, the percentage by weight of the N-long-chain acyl amino acid in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;
or, selecting a primary surfactant consisting of a N-long-chain acyl amino acid, a N-long-chain acyl amino acid dipeptide, and a basic amino acid, wherein preferably, the sum of the percentages by weight of the N-long-chain acyl amino acid and the N-long-chain acyl amino acid dipeptide in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%.

The method according to [26], wherein the cleaning composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a polyol humectant, preferably completely free of a polyol humectant.

The method according to [26] or [27], wherein the preservative in the cleaning composition is benzoic acid.

The method according to any one of [26]-[28], wherein the co-surfactant is substantially free of a nonionic surfactant, preferably, completely free of a nonionic surfactant;

and/or the co-surfactant is substantially free of a cationic surfactant, preferably, completely free of a cationic surfactant;
and/or the co-surfactant is substantially free of an amphoteric surfactant, preferably, completely free of an amphoteric surfactant;
preferably, the percentage by weight of the co-surfactant in the cleaning composition is 0 wt%.

The method according to any one of [26]-[29], wherein the N-long-chain acyl amino acid is lauroyl alanine, the basic amino acid is arginine, and the N-long-chain acyl amino acid dipeptide is lauroyl alanyl alanine.

A method for improving the easy-to-rinse or water-saving performance in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;

the cleaning composition may be the cleaning composition according to any one of [1]-[21];
the method comprises: selecting a primary surfactant consisting of a N-long-chain acyl amino acid and a basic amino acid, wherein preferably, the percentage by weight of the N-long-chain acyl amino acid in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;
or, selecting a primary surfactant consisting of a N-long-chain acyl amino acid, a N-long-chain acyl amino acid dipeptide, and a basic amino acid, wherein preferably, the sum of the percentages by weight of the N-long-chain acyl amino acid and the N-long-chain acyl amino acid dipeptide in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;
or, selecting a primary surfactant consisting of a N-long-chain acyl amino acid and an inorganic base, wherein preferably, the percentage by weight of the N-long-chain acyl amino acid in the cleaning composition is 1-12 wt%, preferably 2-10 wt%, and more preferably 4-8 wt%, and the percentage by weight of the inorganic base in the cleaning composition is 0.1-3 wt%, preferably 0.2-2.5 wt%, and more preferably 0.4-2 wt%.

The method according to [31], wherein the cleaning composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a humectant, preferably completely free of a humectant.

The method according to [31] or [32], wherein the co-surfactant is substantially free of a nonionic surfactant, preferably, completely free of a nonionic surfactant;

and/or the co-surfactant is substantially free of a cationic surfactant, preferably, completely free of a cationic surfactant;
and/or the co-surfactant is substantially free of an amphoteric surfactant, preferably, completely free of an amphoteric surfactant;
preferably, the percentage by weight of the co-surfactant in the cleaning composition is 0 wt%.

The method according to any one of [31]-[33], wherein the N-long-chain acyl amino acid is lauroyl alanine, the basic amino acid is arginine, and the N-long-chain acyl amino acid dipeptide is lauroyl alanyl alanine.

A method for improving the deodorization performance in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;

in some embodiments, the cleaning composition may be the cleaning composition according to any one of [1]-[21];
the method comprises: selecting a primary surfactant consisting of a N-long-chain acyl amino acid and a basic amino acid, wherein the basic amino acid is present in an amount partially neutralizing the N-long-chain acyl amino acid, preferably with the degree of neutralization being 85% or greater and less than 100%, more preferably 88% or greater and 96% or less;
or, selecting a primary surfactant consisting of a N-long-chain acyl amino acid, a N-long-chain acyl amino acid dipeptide, and a basic amino acid, wherein the basic amino acid is present in an amount partially neutralizing the N-long-chain acyl amino acid and the N-long-chain acyl amino acid dipeptide, preferably with the degree of neutralization being 85% or greater and less than 100%, more preferably 88% or greater and 96% or less;
or, selecting a primary surfactant consisting of a N-long-chain acyl amino acid and an inorganic base, wherein the inorganic base is present in an amount partially neutralizing the N-long-chain acyl amino acid, preferably with the degree of neutralization being 85% or greater and less than 100%, more preferably 88% or greater and 96% or less.

The method according to [35], wherein the cleaning composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a humectant, preferably completely free of a humectant.

The method according to [35] or [36], wherein the co-surfactant is substantially free of a nonionic surfactant, preferably, completely free of a nonionic surfactant;

and/or the co-surfactant is substantially free of a cationic surfactant, preferably, completely free of a cationic surfactant;
and/or the co-surfactant is substantially free of an amphoteric surfactant, preferably, completely free of an amphoteric surfactant;
preferably, the percentage by weight of the co-surfactant in the cleaning composition is 0 wt%.

The method according to any one of [35]-[37], wherein the N-long-chain acyl amino acid is lauroyl alanine, the basic amino acid is arginine, and the N-long-chain acyl amino acid dipeptide is lauroyl alanyl alanine.

A method for imparting the oil control performance to a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;

in some embodiments, the cleaning composition may be the cleaning composition according to any one of [1]-[21];
the method comprises: selecting a primary surfactant consisting of a N-long-chain acyl amino acid and a basic amino acid; or selecting a primary surfactant consisting of a N-long-chain acyl amino acid, a N-long-chain acyl amino acid dipeptide, and a basic amino acid; or selecting a primary surfactant consisting of a N-long-chain acyl amino acid and an inorganic base.

The method according to [39], wherein the cleaning composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a humectant, preferably completely free of a humectant.

The method according to [39] or [40], wherein the co-surfactant is substantially free of a nonionic surfactant, preferably, completely free of a nonionic surfactant;

and/or the co-surfactant is substantially free of a cationic surfactant, preferably, completely free of a cationic surfactant;
and/or the co-surfactant is substantially free of an amphoteric surfactant, preferably, completely free of an amphoteric surfactant;
preferably, the percentage by weight of the co-surfactant in the cleaning composition is 0 wt%.

The method according to any one of [39]-[41], wherein the N-long-chain acyl amino acid is lauroyl alanine, the basic amino acid is arginine, and the N-long-chain acyl amino acid dipeptide is lauroyl alanyl alanine.

Compared with the prior art, the present disclosure has the following beneficial technical effects:

1. The use of a N-long-chain acyl amino acid and a basic amino acid/inorganic base as the primary surfactant (especially the use of a basic amino acid) and the control of the amount of the surfactant within a proper range allow the cleaning composition to combine properties of low irritation, selective cleaning/detergency, ease to rinse, no stickiness, etc.
2. The cleaning composition can be substantially free of a nonionic surfactant, a cationic surfactant, and an amphoteric surfactant by selecting a specific primary surfactant, particularly a combination of a N-long-chain acyl amino acid (optionally, a dipeptide) and a basic amino acid.
3. In the present disclosure, it was surprisingly found that benzoic acid has very good preservative performance and compatibility in the system of the present disclosure and is less irritant than other preservatives such as sodium benzoate.
4. In the absence of conventional petroleum-based surfactants, the cleaning composition of the present disclosure still achieves an excellent detergent effect and even selective detergency.
5. In the present disclosure, it was found for the first time that skin irritation or eye irritation can be reduced by selecting a primary surfactant consisting of a N-long-chain acyl amino acid (optionally, a dipeptide) and a basic amino acid without adding a thickening agent or the like.
6. In the present disclosure, it was found that by selecting a specific primary surfactant, preferably controlling the amount of the primary surfactant, and controlling the addition of other components, the ease-to-rinse performance or the water-saving performance can be improved.
7. In the present disclosure, it was surprisingly found that when the degree of neutralization is 85% or greater and less than 100%, the composition system contains a small amount of unneutralized N-long-chain acyl amino acid and/or N-long-chain acyl amino acid dipeptide, which has a very good deodorization effect.
8. In the present disclosure, it was found for the first time that selecting a primary surfactant consisting of a N-long-chain acyl amino acid (optionally, a dipeptide) and a basic amino acid can impart the oil control performance to the cleaning composition.

## BRIEF DESCRIPTION OF THE DRAWINGS

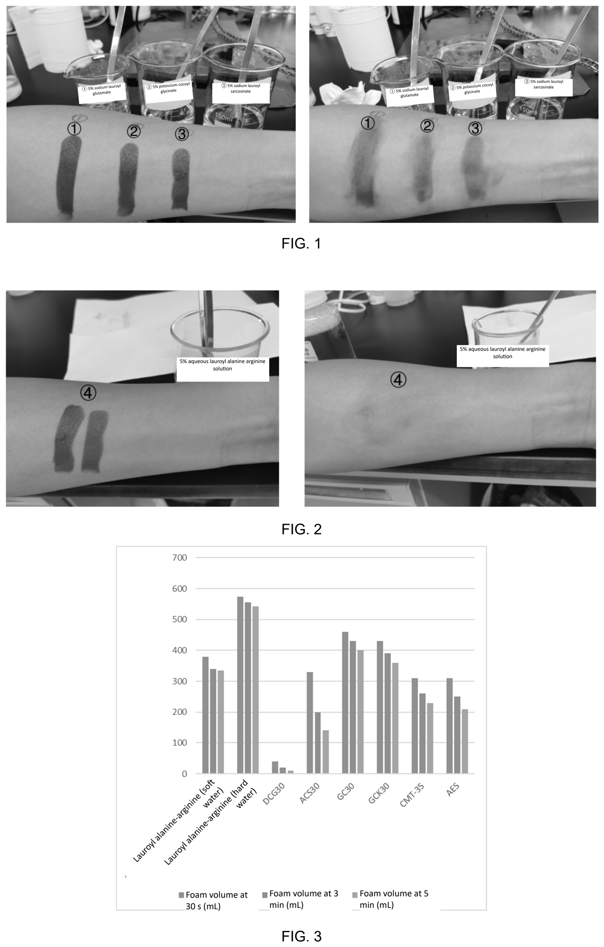

FIG. 1 illustrates the results of the makeup removal test for an aqueous sodium lauroyl glutamate solution, an aqueous potassium cocoyl glycinate solution, and an aqueous sodium lauroyl sarcosinate solution according to Example 4; ① shows the comparison of the aqueous sodium lauroyl glutamate solution before and after treatment; ② shows the comparison of an aqueous potassium cocoyl glycinate solution before and after treatment; ③ shows the comparison of an aqueous sodium lauroyl sarcosinate solution before and after treatment.
FIG. 2 illustrates the results of the makeup removal test for an aqueous lauroyl alanine arginine solution according to Example 4; ④ shows the comparison of the aqueous lauroyl alanine arginine solution before and after treatment.
FIG. 3 illustrates the results of the foam test according to Example 5.

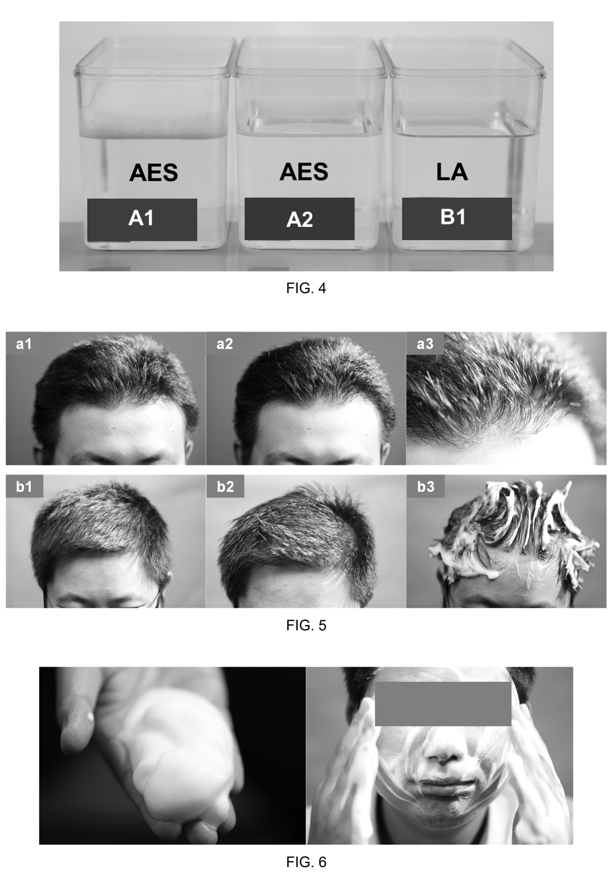

FIG. 4 illustrates the foam condition of the rinsing solution according to Example 7. A1 and A2 are the foam conditions after the first rinse and the second rinse with the cleaning liquid containing AES, respectively, and B1 is the foam condition after the first rinse with the cleaning liquid containing LA.
FIG. 5 illustrates the comparison between the effects of the dry-cleanable shampoo of Example 11 and commercially available shampoos, wherein a1-a3 show the effects of the dry-cleanable shampoo in Example 10, wherein a1 shows the effect before washing, a2 shows the effect after washing and air-drying, and a3 shows an enlarged view after washing; b1-b3 show the effects of the commercially available shampoo, wherein b1 shows the effect before washing, b2 shows the effect after washing and air-drying, and b3 shows the effect after washing and air-drying, moistening the hair with a small amount of water, and kneading.

FIG. 6 illustrates the cleaning effect of the facial cleansing liquid according to Example 12.

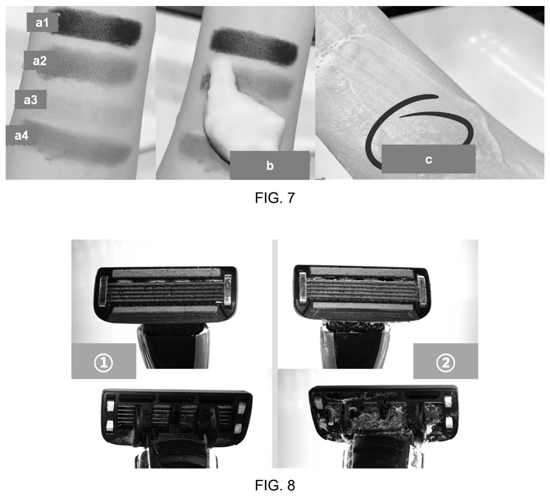

FIG. 7 illustrates the makeup removal effect of the makeup removal cleaning liquid of Example 13, wherein a1 is lipstick, a2 is brow powder, a3 is blusher, and a4 is eye shadow; b shows the effect after applying the makeup removal cleaning liquid in Example 13; c shows the effect after treatment with the makeup removal cleaning liquid in Example 13.

FIG. 8 illustrates the comparison between the shaving cleaning liquid of Example 14 and a commercially available shaving cleaning liquid after use, wherein ① shows the condition of the shaving cleaning liquid of Example 14 remaining on the razor, and ② shows the condition of the commercially available shaving cleaning liquid remaining on the razor.

## DETAILED DESCRIPTION

In one embodiment, provided is a cleaning composition. The composition comprises (a) a primary surfactant, (b) a co-surfactant, and (c) water.

The primary surfactant refers to that in terms of percentage by weight, the surfactant has the highest content of all surfactants; more specifically, the percentage by weight of the primary surfactant in all surfactants is greater than 50 wt%, preferably greater than 65 wt%, and more preferably greater than 80 wt%, for example, greater than 55 wt%, 60 wt%, 65 wt%, 70 wt%, 75 wt%, 80 wt%, 85 wt%, 90 wt%, or 95 wt%. In some examples, the content of the primary surfactant may be up to 100%, that is, no co-surfactant is used.

For component (a), the primary surfactant consists of a N-long-chain acyl amino acid and a basic amino acid. It will be appreciated by those skilled in the art that there is no essential difference between separately adding the N-long-chain acyl amino acid and the basic amino acid to the composition and adding the basic amino acid salt of the N-long-chain acyl amino acid generated in a prior reaction to the composition, and the two compositions are both encompassed within the expression of "consisting of the N-long-chain acyl amino acid and the basic amino acid".

The percentage by weight of the N-long-chain acyl amino acid in the cleaning composition is 1-12 wt%, preferably 4-8 wt%, and more preferably 5-7 wt%, for example, 2 wt%, 3 wt%, 4 wt%, 5 wt% or greater and 11 wt%, 10 wt%, 9 wt%, or 8 wt% or less.

If the content of the N-long-chain acyl amino acid is greater than 12 wt%, the feel of the cleaning composition will gradually become sticky, and if the content of the N-long-chain acyl amino acid is less than 1 wt%, the detergency may be insufficient. To combine the detergency with a good feel, the N-long-chain acyl amino acid is most properly controlled at 4-8 wt%, and more preferably at 5-7 wt%.

The percentage by weight of the basic amino acid in the cleaning composition is 0.5-8 wt%, preferably 1-6.5 wt%, and more preferably 2-5.5 wt%, for example, 1 wt%, 1.5 wt%, 2 wt%, 2.5 wt% or greater and 7 wt%, 6 wt%, 5 wt%, or 4 wt% or less.

The N-long-chain acyl group in the N-long-chain acyl amino acid is derived from a saturated or unsaturated, linear or branched fatty acid with 8-22 carbon atoms. Furthermore, the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl.

Furthermore, the amino acid in the N-long-chain acyl amino acid is derived from one or more of glycine, alanine, glutamic acid, sarcosine, aspartic acid, leucine, isoleucine, valine, threonine, proline, phenylalanine, arginine, or lysine. Furthermore, the amino acid in the N-long-chain acyl amino acid is derived from one or more of alanine, glycine, glutamic acid, sarcosine, arginine, lysine, or (methyl) taurine, preferably alanine, and most preferably L-alanine.

The N-long-chain acyl (methyl) taurine described herein refers to a N-long-chain acyl methyl taurine or a N-long-chain acyl taurine.

As an example, the N-long-chain acyl amino acid may be selected from cocoyl alanine, lauroyl alanine, cocoyl sarcosine, lauroyl sarcosine, lauroyl glutamic acid, cocoyl glutamic acid, oleoyl glutamic acid, cocoyl glycine, lauroyl glycine, stearoyl glutamic acid, and the like, preferably lauroyl alanine and lauroyl sarcosine, and more preferably N-lauroyl-L-alanine.

For the synthesis process of N-long-chain acyl amino acids, one may refer to the general synthesis methods for N-acyl amino acid surfactants, including direct synthesis and indirect synthesis. The direct synthesis methods using fatty acid ingredients include enzymatic synthesis and dehydration synthesis. The indirect synthesis methods include fatty acid chloride acylation, aliphatic nitrile acylation hydrolysis, fatty acid anhydride acylation, amide carbonylation, and the like. One preferred method is the reaction between a fatty acid chloride and the amino group of an amino acid (Shotten-Baumann condensation or Shotten-Baumann reaction).

A typical preparation process suitable for the present disclosure comprises, for example: dissolving an amino acid and sodium hydroxide in water or a mixed solution of water and acetone to give an amino acid salt solution; slowly and dropwise adding lauroyl chloride and a sodium hydroxide solution into the amino acid salt solution, controlling the pH value of the reaction system, and performing post-treatment on the product after the dropwise addition. Representative methods are disclosed in CN1798821A, US6703517B2, CN102875409B, JPH0570418A, etc.

For the post-treatment of the N-long-chain acyl amino acid product, conventional methods such as recrystallization, water rinsing, drying, etc., may be used. One preferred post-treatment comprises: mixing the crude N-long-chain acyl amino acid product with a solvent, optionally stirring, and controlling the temperature T of the mixture system in a range of the melting point of the long-chain fatty acid to the melting point of the N-long-chain acyl amino acid, wherein the solvent is water, an organic solvent, or a mixed solution of water and an organic solvent; and after the temperature control in the system, separating the solid and liquid phases. Related methods are disclosed by the inventor in CN202210867760.7 or PCT/CN2022/107270, the relevant content of which is incorporated herein.

The basic amino acid is selected from one or more of arginine, lysine, citrulline, ornithine, creatine, histidine, diaminobutyric acid, and diaminopropionic acid, preferably arginine and/or lysine, and most preferably arginine.

For component (a), the primary surfactant may also consist of a N-long-chain acyl amino acid, a N-long-chain acyl amino acid dipeptide, and a basic amino acid. The introduction of the N-long-chain acyl amino acid dipeptide can further improve the detergency and foaming performance of the cleaning composition.

The types, synthesis methods, and the like of the N-long-chain acyl amino acid and the basic amino acid have been described above. The N-long-chain acyl amino acid dipeptide, is a known concept, and refers to a long-chain acyl aminoacyl amino acid, typically, for example, long-chain acyl glycyl glycine, long-chain acyl glutamyl glutamic acid, long-chain acyl alanyl alanine, long-chain acyl sarcosyl sarcosine, and the like, as described in, e.g., CN100448968C, PCT/CN2022/107270, etc.

The N-long-chain acyl amino acid dipeptide may be prepared from an acid chloride derived from a N-long-chain acyl amino acid and an amino acid by the Schotten-Baumann reaction or the like, or by the reaction of an amino acid dipeptide (such as glutamyl glutamic acid) and an acid chloride. One preferred relevant preparation method is disclosed in PCT/CN2022/107270, the relevant content of which is incorporated herein.

The N-long-chain acyl group in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl.

The amino acids in the N-long-chain acyl amino acid dipeptide are derived from one or more of alanine, glycine, glutamic acid, sarcosine, arginine, lysine, or (methyl) taurine, preferably L-alanine. A N-long-chain acyl-L-alanine dipeptide, particularly, N-lauroyl-L-alanyl-L-alanine, is preferred in the present disclosure.

The percentage by weight of the N-long-chain acyl amino acid and the N-long-chain acyl amino acid dipeptide in the cleaning composition is 1-12 wt%, preferably 2-10 wt%, more preferably 4-8 wt%, and most preferably 5-7 wt%, for example, 2 wt%, 3 wt%, 4 wt%, 5 wt% or greater and 11 wt%, 10 wt%, 9 wt%, or 8 wt% or less. The percentage by weight of the basic amino acid in the cleaning composition is 0.5-8 wt%, preferably 1-6.5 wt%, and more preferably 2-5.5 wt%, for example, 1 wt%, 1.5 wt%, 2 wt%, 2.5 wt% or greater and 7 wt%, 6 wt%, 5 wt%, or 4 wt% or less.

Furthermore, the percentage by weight of the N-long-chain acyl amino acid dipeptide in the composition is 0.1 wt% or greater, preferably 0.3 wt% or greater, and more preferably 0.5 wt% or greater.

Furthermore, the mass ratio of the N-long-chain acyl amino acid to the N-long-chain acyl amino acid dipeptide is 20:1 to 1.5:1, preferably 15:1 to 3:1, and more preferably 10:1 to 6:1. The mass ratio of the N-long-chain acyl amino acid to the N-long-chain acyl amino acid dipeptide is, for example, 20, 19, 18, 17, 16, 15, 14, 13, 12, 11, 10, 9, 8, 7, 6, 5, 4, or 3.

For component (a), the primary surfactant may also consist of a N-long-chain acyl amino acid and an inorganic base. This case is advantageous in terms of cost-efficiency, but is not the most preferred mode of the present disclosure, as it is inferior to the primary surfactant consisting of the N-long-chain acyl amino acid (optionally, the dipeptide) and the basic amino acid in eye irritation, skin irritation, oil control, moisture retention, and the like.

The types, synthesis methods, and the like of the N-long-chain acyl amino acid have been described above. The inorganic base is selected from one or more of sodium hydroxide and potassium hydroxide, preferably, sodium hydroxide. In general, conventional sodium hydroxide can be used without specific limitation. If a sodium hydroxide solution is used, the amount refers to the amount of sodium hydroxide, rather than the amount of the sodium hydroxide solution.

The percentage by weight of the N-long-chain acyl amino acid in the cleaning composition is 1-12 wt%, preferably 2-10 wt%, and more preferably 4-8 wt%, and the percentage by weight of the inorganic base in the cleaning composition is 0.1-3 wt%, preferably 0.2-2.5 wt%, and more preferably 0.4-2 wt%.

For component (b), the co-surfactant comprises one or more of a nonionic surfactant, a cationic surfactant, an amphoteric surfactant, and a sodium salt, a potassium salt and a TEA salt of a N-long-chain acyl amino acid.

The percentage by weight of the co-surfactant in the cleaning composition is 0-10 wt%, for example, 0 wt%, 1 wt%, 2 wt%, 3 wt%, 4 wt%, 5 wt%, 6 wt%, 7 wt%, 8 wt%, or 9 wt%, preferably 0-5 wt%, and more preferably 0-1 wt%. The co-surfactant should not be present in an excessive amount, preferably in a small amount or absent, or otherwise it may affect the final mildness of the cleaning product to the skin/eyes, wash feel, rinsability performance, and the like.

Examples of the nonionic surfactant include: ester surfactants such as fatty acid glycerides, sorbitan fatty acid esters, and sucrose fatty acid esters; ether surfactants such as alkyl polyethylene glycol and polyoxyethylene alkylphenyl ethers; and alkyl polyglycosides. Nonionic surfactants have good emulsifying and solubilizing effects.

Examples of the cationic surfactant include: alkyl quaternary ammonium salts, ester quaternary ammonium salts, alkyl pyridinium salts, alkyl amine salts, and polymeric cationic surfactants. Cationic surfactants are commonly used in shampoo to increase the softness of the hair.

Examples of the amphoteric surfactant include: sulfobetaine amphoteric surfactants, betaine amphoteric surfactants, and amphoacetate amphoteric surfactants. For example, the amphoteric surfactant may be selected from one or more of cocamidopropyl betaine, lauramidopropyl betaine, disodium cocoamphodiacetate, sodium cocoamphoacetate, sodium lauroamphoacetate, cocoyl betaine, lauryl hydroxysultaine, and lauroamidopropyl hydroxysultaine. The amphoteric surfactant has good foaming, foam-stabilizing, and thickening effects.

The cleaning composition of the present disclosure can be substantially free of a nonionic surfactant, a cationic surfactant, and an amphoteric surfactant, preferably completely free of a nonionic surfactant, a cationic surfactant, and an amphoteric surfactant, by selecting a specific primary surfactant, particularly a combination of a N-long-chain acyl amino acid (or a dipeptide) and a basic amino acid, such that the product can have a more pleasant feel, ease to rinse, and low irritation. This compounding breaks through the existing formulation theory and achieves surprising effects of refreshing wash feel, ease to rinse, low irritation, and selective detergency.

When a N-long-chain acyl amino acid (optionally, a dipeptide) and a basic amino acid are selected as the primary surfactant, a sodium salt, a potassium salt, and a TEA salt of the corresponding N-long-chain acyl amino acid can be used as the co-surfactant (the definition of the N-long-chain acyl amino acid has been described above), such that the performance and cost-efficiency can be combined. However, regardless of the cost-efficiency, a surfactant composed entirely of a N-long-chain acyl amino acid (or a dipeptide) and a basic amino acid without the use of any co-surfactant is most preferred in the present disclosure.

Component (c) is not specified. Specifically, ion-exchanged water, distilled water, purified water, river water, well water, natural water, underground water, city water, hard water, soft water, or the like may be used. One of these may be used, or two or more selected from the above group may be used in combination. From the viewpoint of storage stability and hygiene of the product of the present disclosure, ion-exchanged water, distilled water, and purified water are preferred.

The cleaning composition may further comprise component (d), a preservative. In the present disclosure, it was surprisingly found that benzoic acid has very good preservative performance in the system of the present disclosure and is less irritant than other preservatives such as sodium benzoate. Therefore, benzoic acid is particularly preferred as the preservative. In general, sodium benzoate is often used in cosmetics due to its better water solubility, whereas in the present system, the combination of the basic amino acid and the N-long-chain acyl amino acid (optionally, a dipeptide) surprisingly solubilizes benzoic acid, thus making the application of benzoic acid in the system possible.

The cleaning composition may further comprise component (e), a perfume or a fragrance. Particularly preferred in the present disclosure is a perfume or a fragrance with good water solubility. It was found that when the primary surfactant is a combination of a N-long-chain acyl amino acid (optionally, a dipeptide) and a basic amino acid, compared with sodium or potassium salts of the N-long-chain acyl amino acid, the primary surfactant provides more outstanding solubilization for perfumes or fragrances, and it is easier to make the solution clear and transparent, which is very important especially when the composition does not contain a polyol.

The cleaning composition may further comprise component (f), hyaluronic acid or sodium hyaluronate, so as to give full play to hyaluronic acid/sodium hyaluronate for moisturizing, repairing skin barrier, alleviating inflammatory responses, and the like.

In addition, the composition of the present disclosure may optionally further comprise an additional ingredient, such as a dye, a chelating agent, an extract, an amino acid, a nucleic acid, a vitamin, an enzyme, an anti-inflammatory agent, a bactericide, an antioxidant, an ultraviolet absorber, an antiperspirant, a pH regulator, a pearlizing agent, etc., which shall not be contradictory to the safety and environment conservation, low irritation (to skin/eyes), ease to rinse, refreshing wash feels, oil control, and other objectives pursued in the present disclosure.

Thickening agent: In existing cleaning products, thickening agents are often added to give liquids with a certain viscosity. Common thickening agents include cellulose thickening agents, mucopolysaccharide thickening agents, polyvinyl alcohol thickening agents, colloid thickening agents, sodium polyacrylate thickening agents, polyvinyl pyrrolidone thickening agents, vinyl polymers thickening agents, etc. For example, common thickening agents are starch, gelatin, sodium alginate, guar gum, chitin gum, gum arabic, xanthan gum, soy protein gum, polyacrylamide, polyvinyl alcohol, polyvinylpyrrolidone, modified paraffin resin, carbomer resin, styrene butadiene rubber, or the like. However, the addition of such thickening agents may increase the irritations of the product to skin and eyes and destroy the refreshing wash feel of the product. Therefore, the cleaning composition of the present disclosure is preferably substantially free of a thickening agent, and more preferably, completely free of a thickening agent.

Sulfate surfactant: Examples include sodium lauryl sulfate (SLS), ammonium lauryl sulfate (ALS), sodium laureth sulfate (SLES), or ammonium laureth sulfate (ALES). Considering the potential irritation to the skin or scalp and/or damage to the hair, the cleaning composition of the present disclosure is preferably substantially free of a sulfate surfactant, and more preferably, completely free of a sulfate surfactant.

Ethoxylate: The representative is sodium laureth sulfate (SLES or AES). Considering its moderate irritation, the large amount of non-renewable carbon from ethylene oxide in ethoxylate surfactants, and safety concerns (the carcinogenicity of impurity dioxane), the cleaning composition of the present disclosure is preferably substantially free of an ethoxylate, and more preferably, completely free of an ethoxylate.

Fatty acid salt: Examples include laurate, myristate, palmitate, and stearate. In view of the after-wash tension, dryness, and tightness caused by such ingredients, the cleaning composition of the present disclosure is preferably substantially free of a fatty acid salt, and more preferably completely free of a fatty acid salt.

Polyol humectant: Examples include glycerols such as glycerol, diglycerol, and polyglycerol, sugar alcohols such as sorbitol, erythritol, and xylitol, and glycols such as isoprene glycol, 1,2-propanediol (PG), dipropylene glycol (DPG), ethoxydiglycol, and 1,3-butanediol (BG). **The** addition of polyols may bring a heavily sticky feel, and the cleaning composition of the present disclosure is preferably substantially free of a polyol humectant, and more preferably completely free of a polyol humectant. Generally, polyol humectants are required in cleaning products. However, by adopting a N-long-chain acyl amino acid (optionally, a dipeptide) and a basic amino acid as the primary surfactants, the present disclosure surprisingly solves the problems of moisture retention, solubilization, and the like while exerting the cleaning effect.

In one embodiment, provided is a method for reducing the skin irritation or eye irritation in a cleaning composition, wherein the cleaning composition comprises: (a) a primary surfactant, (b) a co-surfactant, (c) water, and optionally, an additional ingredient. Components (a), (b), (c) and other optional components have been described in other embodiments and are not recited herein.

The method comprises: selecting a primary surfactant consisting of a N-long-chain acyl amino acid and a basic amino acid, wherein preferably, the percentage by weight of the N-long-chain acyl amino acid in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;

or, selecting a primary surfactant consisting of a N-long-chain acyl amino acid, a N-long-chain acyl amino acid dipeptide, and a basic amino acid, wherein preferably, the sum of the percentages by weight of the N-long-chain acyl amino acid and the N-long-chain acyl amino acid dipeptide in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%.

From the viewpoint of reducing irritation, it is preferred that the composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a polyol humectant, preferably completely free of a polyol humectant.

The preservative benzoic acid has low irritation, so in order to reduce the skin irritation or eye irritation, it is preferred to use benzoic acid as a preservative in the cleaning compositions.

The proper pH range for the human body is weakly acidic. Therefore, in order to reduce the skin irritation or eye irritation, the pH of the cleaning composition is preferably 5.5-6.9, more preferably 6-6.7. Illustratively, the proper pH may be 5.5, 5.6, 5.7, 5.8, 5.9, 6.0, 6.1, 6.2, 6.3, 6.4, 6.5, 6.6, 6.7, 6.8, or 6.9.

Furthermore, the cleaning composition is substantially free of a nonionic surfactant, a cationic surfactant, and an amphoteric surfactant. Still furthermore, the percentage by weight of the co-surfactant in the cleaning composition is 0 wt%.

Furthermore, the N-long-chain acyl amino acid is lauroyl alanine, the basic amino acid is arginine, and the N-long-chain acyl amino acid dipeptide is lauroyl alanyl alanine. That is, the primary surfactant is selected to consist of a N-long-chain acyl amino acid and a basic amino acid, wherein the N-long-chain acyl amino acid is lauroyl alanine and the basic amino acid is arginine; or the primary surfactant is selected to consist of a N-long-chain acyl amino acid, a N-long-chain acyl amino acid dipeptide, and a basic amino acid, wherein the N-long-chain acyl amino acid is lauroyl alanine, the N-long-chain acyl amino acid dipeptide is lauroyl alanyl alanine, and the basic amino acid is arginine.

In one embodiment, provided is a method for improving the easy-to-rinse or water-saving performance in a cleaning composition, wherein the cleaning composition comprises: (a) a primary surfactant, (b) a co-surfactant, (c) water, and optionally, an additional ingredient. Components (a), (b), (c) and other optional components have been described in other embodiments and are not recited herein.

The term "ease-to-rinse performance" or "water-saving performance" refers to that when products such as facial cleanser, facial mousse, and body wash are used, they can be quickly removed by rinsing with a small amount of water and provide a refreshing and non-sticky feel after rinsing. As reported, the ease-to-rinse property may be attributed to that during the rinsing, the surfactant binds to cations such as calcium ions and the like to form precipitates or does not easily form micelles in water, leading to a very low amount of the surfactant adsorbed on the skin during the rinsing and thus ease to rinse. On the contrary, if the surfactant does not bind to the cations or is present in the system as micelles, a greater amount of anionic surfactant is adsorbed on the skin during the rinsing, which is represented by a slippery feel on the skin during the rinsing, resulting in an "illusion" of incomplete rinsing in use, thereby prolonging the rinsing time and consuming more water.

By selecting a specific primary surfactant and controlling the amount of the primary surfactant, the ease-to-rinse or water-saving performance can be improved. The method comprises: selecting a primary surfactant consisting of a N-long-chain acyl amino acid and a basic amino acid, wherein preferably, the percentage by weight of the N-long-chain acyl amino acid in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;

or, selecting a primary surfactant consisting of a N-long-chain acyl amino acid, a N-long-chain acyl amino acid dipeptide, and a basic amino acid, wherein preferably, the sum of the percentages by weight of the N-long-chain acyl amino acid and the N-long-chain acyl amino acid dipeptide in the cleaning composition is 1-12 wt%, preferably 2-10 wt%, and more preferably 4-8 wt%, and the percentage by weight of the basic amino acid in the cleaning composition is 0.5-8 wt%, preferably 1-6.5 wt%, and more preferably 2-5.5 wt%;

or, selecting a primary surfactant consisting of a N-long-chain acyl amino acid and an inorganic base, wherein preferably, the percentage by weight of the N-long-chain acyl amino acid in the cleaning composition is 1-12 wt%, preferably 2-10 wt%, and more preferably 4-8 wt%, and the percentage by weight of the inorganic base in the cleaning composition is 0.1-3 wt%, preferably 0.2-2.5 wt%, and more preferably 0.4-2 wt%.

For the N-long-chain acyl amino acid/salt, an excessive amount of the N-long-chain acyl glutamate may lead to an unsatisfactory foaming property and impart a sticky feel to the product, resulting in low rinsability. N-long-chain acyl glycinates are more refreshing than N-long-chain acyl glutamates in terms of wash feel. However, N-long-chain acyl glycinates are prone to precipitate in weakly acidic to neutral systems, resulting in system instability. The N-long-chain acyl methyl taurate residue also leads to a sticky feel. Therefore, from the viewpoint of improving the ease-to-rinse or water-saving performance, the N-long-chain acyl amino acid is particularly preferably lauroyl alanine due to its good and non-sticky wash feel and stability under weakly acidic conditions.

The basic amino acid, particularly arginine, has a certain complexing effect on calcium and magnesium ions in water, and can improve the ease-to-rinse performance when used in combination with a N-long-chain acyl amino acid as the primary surfactant.

Most preferably, the primary surfactant is selected to consist of lauroyl alanine and arginine, or the primary surfactant is selected to consist of lauroyl alanine, lauroyl alanyl alanine, and arginine.

In addition, from the viewpoint of improving the ease-to-rinse or water-saving performance, it is preferred that the composition is substantially free of a thickening agent, preferably completely free of a thickening agent; and/or substantially free of a sulfate, preferably completely free of a sulfate; and/or substantially free of an ethoxylate, preferably completely free of an ethoxylate; and/or substantially free of a fatty acid salt, preferably completely free of a fatty acid salt; and/or substantially free of a polyol humectant, preferably completely free of a polyol humectant.

Furthermore, the cleaning composition is substantially free of a nonionic surfactant, a cationic surfactant, and an amphoteric surfactant. Still furthermore, the percentage by weight of the co-surfactant in the cleaning composition is 0 wt%.

In one embodiment, provided is a cleaning composition having excellent deodorization performance, comprising: (a) a primary surfactant, (b) a co-surfactant, (c) water, and optionally, an additional ingredient. Components (a), (b), (c) and other optional components have been described in other embodiments. Meanwhile, the present disclosure further provides a method for improving the deodorization performance in a cleaning composition.

The method comprises: selecting a primary surfactant consisting of a N-long-chain acyl amino acid and a basic amino acid, wherein the basic amino acid is present in an amount partially neutralizing the N-long-chain acyl amino acid, preferably with the degree of neutralization being 85% or greater and less than 100%, more preferably 88% or greater and 96% or less;

or, selecting a primary surfactant consisting of a N-long-chain acyl amino acid, a N-long-chain acyl amino acid dipeptide, and a basic amino acid, wherein the basic amino acid is present in an amount partially neutralizing the N-long-chain acyl amino acid and the N-long-chain acyl amino acid dipeptide, preferably with the degree of neutralization being 85% or greater and less than 100%, more preferably 88% or greater and 96% or less;

or, selecting a primary surfactant consisting of a N-long-chain acyl amino acid and an inorganic base, wherein the inorganic base is present in an amount partially neutralizing the N-long-chain acyl amino acid, preferably with the degree of neutralization being 85% or greater and less than 100%, more preferably 88% or greater and 96% or less.

In one preferred embodiment, the degree of neutralization is 85% or greater. For example, the degree of neutralization is 86%, 87%, 88%, 89%, 90%, 91%, 92%, 93%, 94%, 95%, 96%, 97%, or 98%. Furthermore, the molar ratio of the N-long-chain acyl amino acid (optionally, the dipeptide) to the basic amino acid/inorganic base is 1:0.98 to 1:0.85, preferably 1:0.96 to 1:0.88, and more preferably 1:0.95 to 1:0.90. Illustratively, when the N-long-chain acyl amino acid is present at 1 mol, the basic amino acid is present at 0.98, 0.97, 0.96, 0.95, 0.94, 0.93, 0.92, 0.91, 0.90, 0.89, 0.88, 0.87, 0.86, or 0.85 mol.

In the present disclosure, it was surprisingly found that when the degree of neutralization is 85% or greater and less than 100%, the composition system contains a small amount of unneutralized N-long-chain acyl amino acid and/or N-long-chain acyl amino acid dipeptide, which has a very good deodorization effect.

Lauroyl alanine molecules easily self-assemble to form a supramolecular structure, which facilitates the adsorption and removal of odor molecules. Therefore, most preferably, the N-long-chain acyl amino acid is lauroyl alanine (N-lauroyl-L-alanine), the basic amino acid is arginine (L-arginine), and the N-long-chain acyl amino acid dipeptide is lauroyl alanyl alanine (N-lauroyl-L-alanyl-L-alanine).

In one embodiment, provided is a method for imparting the oil control performance to a cleaning composition, wherein the cleaning composition comprises: (a) a primary surfactant, (b) a co-surfactant, (c) water, and optionally, an additional ingredient. Components (a), (b), (c) and other optional components have been described in other embodiments and are not recited herein.

The method comprises: selecting a primary surfactant consisting of a N-long-chain acyl amino acid and a basic amino acid; or selecting a primary surfactant consisting of a N-long-chain acyl amino acid, a N-long-chain acyl amino acid dipeptide, and a basic amino acid; or selecting a primary surfactant consisting of a N-long-chain acyl amino acid and an inorganic base.

In the present disclosure, it was found for the first time that the combination of a N-long-chain acyl amino acid (optionally a dipeptide) and a basic amino acid possesses the optimal oil control performance.

Most preferably, the N-long-chain acyl amino acid is lauroyl alanine, the basic amino acid is arginine, and the N-long-chain acyl amino acid dipeptide is lauroyl alanyl alanine.

In the above embodiments, the composition of the present disclosure can be further controlled in a range from slightly acidic to slightly basic, preferably slightly acidic. That is, the pH of the composition is 5-7.5, preferably 5.5-6.9, and more preferably 6-6.7. Illustratively, the proper pH may be 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, or 7.5.

In the above embodiments, the composition of the present disclosure further possesses a low viscosity. The low viscosity refers to a viscosity at 25 °C of less than 150 mPa·s, preferably less than 120 mPa·s, and more preferably less than 100 mPa·s. The lower limit of the viscosity is not specified, and examples thereof include 1 mPa·s or higher. The viscosity can be measured by a conventional method. For example, a rotational viscometer can be used to measure the composition at 25 °C.

In the above embodiments, the composition of the present disclosure further exhibits selective detergency performance. That is, the JB-01 and JB-02 have higher values, while the JB-03 has a lower value, as measured by the test method in QB/T1224-2012 Liquid Detergent for Fabric. Furthermore, the JB-01 is 0.8 or higher, preferably 0.81, 0.82, 0.83, 0.84, 0.85, 0.86, 0.87, 0.88, 0.89, or 0.9 or higher; the JB-02 is 0.8 or higher, preferably 0.81, 0.82, 0.83, 0.84, 0.85, 0.86, 0.87, 0.88, 0.89, or 0.9 or higher; the JB-03 is 0.4 or higher and 0.8 or lower, preferably 0.45, 0.46, 0.47, 0.48, 0.49, 0.50, 0.51, 0.52, 0.53, 0.54, or 0.55 or higher and 0.79, 0.78, 0.77, 0.76, 0.75, 0.74, 0.73, 0.72, 0.71, 0.70, 0.69, 0.68, 0.67, 0.66, or 0.65 or lower.

Conventional petroleum-based surfactants such as AES may exhibit excessive lipid cleaning effects on the human skin (up to 1.4 for JB-03), destroy the protective barrier on the skin, and result in an excessive loss of sebum, and harmful substances may easily invade or irritate the skin, thereby causing dry, tight, and prickling feels on the skin and even sensitivity after washing. The composition of the present disclosure (particularly the combination of the N-long-chain acyl amino acid and the basic amino acid) can selectively remove dirt without excessively cleaning "squalene", triglyceride and the like that protect the skin, thereby protecting the skin moisture barrier (JB-01 and JB-02 exceed 0.8 or even 0.9, while JB-03 is kept around 0.5).

In the above embodiments, the composition of the present disclosure further exhibits unexpectedly excellent hard water resistance. Furthermore, the composition has better foaming performance in hard water than in soft water as per the test standard GB/T7462-1994 "Surfactant: Measurement of Foaming Performance-Modified Ross-Miles Method" on a 2152 modified Ross-Miles foaming tester. Preferably, the foam volumes at 30 s, 3 min, and 5 min in hard water are greater than 400 mL.

Most preferably, the primary surfactant is selected to consist of lauroyl alanine (optionally a dipeptide) and arginine, wherein the percentage by weight of lauroyl alanine in the cleaning composition is 1-12 wt%, preferably 2-10 wt%, and more preferably 4-8 wt%, and the percentage by weight of arginine in the cleaning composition is 0.5-8 wt%, preferably 1-6.5 wt%, and more preferably 2-5.5 wt%. In this case, the foam volumes at 30 s, 3 min, and 5 min in hard water are greater than 450 mL, and more preferably greater than 460 mL, 470 mL, 480 mL, 490 mL, or 500 mL.

The compositions of the present disclosure may be used for preparing a body wash, a shampoo, a facial mousse, a makeup remover, a mouth wash, a shaving product, a hand sanitizer, a cleaning lotion, or a multi-functional cleaning product. The multi-functional cleaning product refers to a cleaning product with multiple uses, such as a two-in-one product for shampoo and body wash, and a three-in-one product for facial wash, shampoo, and body wash.

The composition of the present disclosure may be used for preparing a no-rinse cleaning product or a dry-cleanable cleaning product. The "no-rinse cleaning product" and "dry-cleanable cleaning product" refer to that after the cleaning product is used to remove dirt from the body, hair and other parts, it can be wiped off simply with a dry towel or a wet towel without rinsing with water.

The composition of the present disclosure keeps low irritation while maintaining good detergency, and also imparts certain moisturizing performance to the product when the combination of arginine and the N-long-chain acyl amino acid is selected as the primary surfactant. Thus, the composition is highly suitable for use in treating or cleaning dry, aged, or damaged skin, or in intimate care, particularly suitable for use in fabricating a cleaning product for the elderly.

In the embodiments, wherever the details, type selection, amount selection, and the like that are not discussed do not conflict with the content, reference may be made to the related teachings in other embodiments, which will not be recited for brevity.

The "substantially free of" described in the present disclosure refers to that the content of the substance in the composition does not significantly and substantially affect the performance of the product. Furthermore, the "substantially free of" refers to that the percentage by weight of the substance in the composition is less than 0.5 wt%, preferably less than 0.1 wt% or less than 0.01 wt%, and more preferably less than the limit of detection.

Unless otherwise specified, the representations "or higher", "or lower", and "A-B" in the present disclosure are all inclusive. The amount refers to the amount of a particular substance, rather than the amount of the corresponding solution (if any).

The combination of the N-long-chain acyl amino acid (optionally, the dipeptide) and the basic amino acid described herein refers to that the primary surfactant consists of the N-long-chain acyl amino acid and the basic amino acid, or the primary surfactant consists of the N-long-chain acyl amino acid, the N-long-chain acyl amino acid dipeptide, and the basic amino acid.

The present disclosure will be further illustrated by the following examples in conjunction with the accompanying drawings. It will be appreciated that these examples are merely intended to illustrate the present disclosure rather than limit the scope of the present disclosure. Furthermore, it will be appreciated that various changes or modifications of the present disclosure can be made by those skilled in the art after reading the teachings of the present disclosure, and such equivalents also fall within the scope of the appended claims of the present application.

The present disclosure will be further illustrated with reference to the following specific examples.

**Example 1** Skin irritation test

Basis: In Vitro Skin Irritation: Reconstructed Human Epidermis Test Method, OECD TG 439 (2021).

Table 1

| | OD value | | Viability | | | IL-1$\alpha$ content (pg/mL) |
|---|---|---|---|---|---|---|
| | Mean | SD | Mean | SD | CV | |
| Negative control (NC)*1 | 1.1376 | 0.0354 | **100.00%** | 3.11% | 3.1% | 3.9±1.98 |
| Positive control (PC)*2 | 0.0458 | 0.0023 | **4.03%** | 0.20% | 5.0% | 89.45±10.06 |
| LA-NaOH*3 | 0.9895 | 0.0427 | **86.99%** | 3.75% | 4.3% | 3.88±5.28 |
| LA-Arg*4 | 1.1080 | 0.0359 | **97.40%** | 3.16% | 3.2% | 3.5±0.38 |
| Lauroyl alanine (LA) | 1.0866 | 0.0709 | **95.52%** | 6.23% | 6.5% | 2.07±0.59 |
| Sodium laureth sulfate (AES) | 0.6498 | 0.0748 | **57.12%** | 6.58% | 11.5% | 20.59±5.27 |
| Sodium dodecyl sulfate (K12) | 0.4640 | 0.0140 | **40.79%** | 1.23% | 3.0% | 96.26±3.21 |

1. The negative control was a DPBS solution.
2. The positive control was 5% SDS.
3. A salt prepared by the reaction of lauroyl alanine with sodium hydroxide in a molar ratio of 1:1.
4. A salt prepared by the reaction of lauroyl alanine with arginine in a molar ratio of 1:1.

According to the data in Table 1, it can be found that the surfactant based on a N-long-chain acyl amino acid (lauroyl alanine) exhibited a significantly reduced skin irritation, and in particular, the combination of lauroyl alanine with arginine exhibited almost no irritation to human epidermis.

However, conventional surfactants such as AES and K12 exhibited significantly higher irritations, and the addition of these surfactants will adversely affect the mildness and low irritation of the end products.

**Example 2** Eye irritation test

Basis: Bovine Corneal Opacity and Permeability Test, OECD TG 437 (2020).

Table 2

| Sample | Test concentration | OD value | In vitro irritancy score (IVIS) |
|---|---|---|---|
| Cleaning composition (LA-Na)*1 | 10% | 0.6778 | 15.0563 |
| Cleaning composition (LA-Arg) *2 | 10% | 0.5759 | 9.8918 |
| Cleaning composition (AES)*3 | 10% | 0.8553 | 12.5406 |
| Cleaning composition (K12)*4 | 10% | 1.3685 | 25.2809 |
| Negative control (ultrapure water): | / | 0.04997 | 0.9369 |
| Positive control (absolute ethanol): | / | 0.9744 | 32.3545 |

1. The composition consisted of lauroyl alanine, sodium hydroxide, and water, and the content of lauroyl alanine sodium salt was 10%.
2. The composition consisted of lauroyl alanine, arginine, and water, and the content of lauroyl alanine arginine salt was 10%.
3. 1 g of AES (70%) was diluted to 10 mL (10% (W/V)) with water.
4. 1 g of K12 was diluted to 10 mL (10% (W/V)) with water.

According to the data in Table 2, it can be found that the surfactant containing the combination of a N-long-chain acyl amino acid and a basic amino acid (the combination of lauroyl alanine and arginine) was the least irritating to eyes. However, conventional surfactants such as AES and K12 and the sodium salt of a N-long-chain acyl amino acid (sodium salt of lauroyl alanine) all exhibited relatively high irritations to eyes.

**Example 3** Detergency test

Basis: QB/T 1224-2012 Liquid Detergent for Fabric. The following contents are percentages by weight.

JB-01 relates to artificial mixed oil stains, mainly verifying the detergency to oil and dust stains; JB-02 relates to various protein stains, mainly verifying the detergency to protein stains; JB-03 relates to artificial sebum stains, mainly verifying the detergency to human oil stains.

Table 3

| Sample | JB-01 | JB-02 | JB-03 |
|---|---|---|---|
| 10% lauroyl alanine + 6.4% arginine, the balance of water | 0.91 | 0.98 | 0.55 |
| 10% lauroyl alanine + 1.46% sodium hydroxide, the balance of water | 0.94 | 0.81 | 0.64 |
| 10% sodium cocoyl glutamate, the balance of water | 0.82 | 0.81 | 0.60 |
| 10% sophorolipid, the balance of water | 0.70 | 0.70 | 0.77 |
| 10% AES, the balance of water | 0.97 | 0.72 | 1.40 |
| 10% K12, the balance of water | 0.98 | 0.80 | 0.93 |

According to the data in Table 3, it can be found that the values of JB-01 and JB-02 were relatively high when lauroyl alanine was compounded with either an inorganic base (sodium hydroxide in this example) or a basic amino acid (arginine in this example), suggesting good capability of removing oil, dust, and protein stains; the value of JB-03 was moderate, suggesting moderate capability of removing sebum stains of the human body and a significantly selective capability of removing stains. This is very important to protect the skin moisture barrier without excessive cleaning of the skin's "squalene", triglycerides, and the like.

In addition, the data also demonstrate that the surfactant based on lauroyl alanine-arginine has a very good detergency for oil, dust, and protein stains, which is significantly greater than those of common mild surfactants such as glutamate, sophorolipid, etc., making it possible to use lauroyl alanine-arginine as the primary surfactant.

**Example 4** Makeup removal test

Reagents: ① aqueous sodium lauroyl glutamate solution, ② aqueous potassium cocoyl glycinate solution, ③ aqueous sodium lauroyl sarcosinate solution, and ④ aqueous lauroyl alanine arginine solution.

Procedures: 4 commercially available amino acid surfactant solutions were mixed with water in a ratio of 1:20 to prepare standard aqueous solutions; 2 g of each sample was applied to a lipstick-applied area on the skin, and then wiped by hand after the skin was wet for inspecting the makeup removal capacity.

The solid contents of the commercially available sodium lauroyl glutamate solution, potassium cocoyl glycinate solution, and sodium lauroyl sarcosinate solution are about 30%-35%. The lauroyl alanine arginine solution was LA-Arg 30 (containing 20% lauroyl alanine + 12.8% arginine) supplied by Suzhou Weimei Biotech Co., Ltd.

FIGs. 1 and 2 illustrate the removal effects on lipsticks when the skin was wet and then wiped by hand. As can be seen from the drawings, the descending order of the makeup removal capacity was as follows: ④ aqueous lauroyl alanine arginine solution > ② aqueous potassium cocoyl glycinate solution > ③ aqueous sodium lauroyl sarcosinate solution > ① aqueous sodium lauroyl glutamate solution.

It can be seen from this test that the lauroyl alanine-arginine-based surfactant has higher detergency than conventional amino acid surfactants. With reference to Examples 3 and 4, it was verified for the first time that the lauroyl alanine-arginine compounding formulation can be used as the primary surfactant.

**Example 5** Foaming test

Standard: GB/T7462-1994 "Surfactant: Measurement of Foaming Performance-Modified Ross-Miles Method".

Instrument: 2152 modified Ross-Miles foaming tester.

Procedures: standard GB/T7462-1994; the foaming performance was determined and the differences were compared.

Conditions: test solution concentration: 2.5 g/L; test temperature: $50 \pm 0.5$ °C; test water: soft water/hard water.

Reagents: DCG30: sodium cocoyl glutamate; ACS30: sodium cocoyl alaninate; GC30: sodium cocoyl glycinate; GCK30: potassium cocoyl glycinate; SCMT-35: sodium cocoylmethyl taurate; AES: sodium laureth sulfate.

Table 4

| | Test sample | | Outflow time | Foam volume at 30 s (mL) | Foam volume at 3 min (mL) | Foam volume at 5 min (mL) |
|---|---|---|---|---|---|---|
| | Lauroyl alanine-arginine (soft water) | Replicate 1 | 2 min and 14 s | 380 | 340 | 335 |
| | Lauroyl alanine- | Replicate 1 | 2 min and 11 s | 570 | 540 | 530 |
| | | Replicate 2 | 2 min and 13 s | 570 | 560 | 545 |
| | arginine (hard water) | Replicate 3 | 2 min and 13 s | 580 | 565 | 550 |
| Soft water | DCG30 | Replicate 1 | | 40 | 20 | 10 |
| | ACS30 | Replicate 1 | | 330 | 200 | 140 |
| | GC30 | Replicate 1 | | 460 | 430 | 400 |
| | GCK30 | Replicate 1 | | 430 | 390 | 360 |
| | CMT-35 | Replicate 1 | | 310 | 260 | 230 |
| | AES | Replicate 1 | | 310 | 250 | 210 |

The test results are shown in Table 4 and FIG. 3. It can be seen that lauroyl alanine-arginine exhibited better foaming performance in hard water than in soft water, indicating a property of resisting hard water. Considering that water in most parts of China is hard water, lauroyl alanine-arginine is very suitable as the primary surfactant of shampoos, as its foaming performance in hard water is even superior to that of AES.

**Example 6** Deodorization performance

Instrument: Front-loading washing machine (XQB80-Z1708), electronic balance (PL2002), and pipette (5 mL).

Materials and reagents:

JB-00 white cotton cloth (GB/T 13174, 25 cm × 25 cm); artificial sweat (QB/T 1901.2-93); 100% cotton T-shirt; yellow croaker.

Procedures

Fishy smell deodorization assessment: Frozen yellow croakers were placed into a sealed bag, before 30 mL of tap water was added. The bag was left to stand at room temperature for 4 h. Five assessors were requested to wash their hands with clean water to ensure that they had no odors. 5 mL of the water in the sealed bag was dropped in the palm of each assessor. The water with fishy smell was evenly applied on the backs and palms of the assessors' hands. After 5 min, the hands were washed with 2 mL of the test sample solution for 2 min to simulate the hand sanitizer, then washed with tap water, and dried. Whether there was still an odor on the hands was judged by the assessors.

Sweat stain removal assessment: A piece of JB-00 white cotton cloth was evenly dripped with 2 mL of artificial sweat, placed in a sealed bag, and left to stand for 4 h. Three 100% cotton T-shirts and the white cotton cloths with sweat were washed with 60 g of the test sample solution in a washing machine in a common washing mode, with settings of 30 L of water, 2 rinsing procedures, and a 5-min drying procedure. After the washing, the 100% cotton T-shirts and the white cotton cloths were dried in air. Five assessors were requested to judge whether there was any smell on the white cotton cloths.

The assessment was based on the following criteria:
Score 0 - no odor;
Score 1 - mild odor;
Score 2 - remarkable odor.

Table 5

| Test sample solution | Deodorization (average of scores from 5 assessors) Fishy smell score | Deodorization (average of scores from 5 assessors) Sweat smell score |
|---|---|---|
| 1# | 1.8 | 2 |
| 2# | 0.4 | 0.6 |
| 3# | 0 | 0 |
| 1#, lauroyl sarcosinate salt 6% + benzoic acid 0.4% + fragrance 0.25% + balance of water (TO 100) 2#, lauroyl alanine 6% + arginine 3.84% + benzoic acid 0.2% + fragrance 0.25% + balance of water (TO 100) 3#, lauroyl alanine 6% + arginine 3.45% + benzoic acid 0.2% + fragrance 0.25% + balance of water (TO 100) | | |

According to the test data in Table 5, it can be learned that the combination of lauroyl alanine and a basic amino acid in a molar ratio of 1:0.9 exhibited the best deodorization performance.

**Example 7** Ease-to-rinse performance

Test I

Materials and reagents:

a) test sample solutions; b) artificial dirt, prepared as per B2.4 in GB/T 9985-2000; c) anhydrous calcium chloride, AR.; d) magnesium chloride ($MgCl_2 \cdot 6H_2O$), AR.; and e) deionized water.

Instruments and equipment:

a) electronic balance with a precision of 0.01 g; b) 2 white porcelain dishes with an outer diameter of about 250 mm; c) washbasin with a volume of 6 L; d) measuring cylinder, 1000 mL; e) bottle with a lower outlet (see GB/T 9985), 5000 mL; and f) bristle brush, 102 mm.

Procedures:

a) Artificial dirt prepared as per the procedures in B2.5 of GB/T 9985-2000 was applied to two plates in an amount of 4 g/plate, and the plates were left to stand at room temperature overnight for later use;
b) The two test plates were washed as per the washing procedures in B2.6 of GB/T 9985-2000 (with the test sample solutions as the test sample);
c) 1000 mL of deionized water was transferred into a bottle with a lower outlet (the lower part of the outlet tube of the bottle was prefilled with deionized water and the water was discharged until no more water came out), the outlet was then opened to rinse the plate from the step (b) with 1000 mL of deionized water, and the rinsings were collected.
d) Procedure (c) was repeated for each test plate to give second rinsings and third rinsings.

Assessment of results:

The pH values of the obtained rinsings were measured to determine the residue of the test sample, and the results were used to determine the ease-to-rinse performance of the product.

Table 6

|  | Conclusion |
|---|---|
| 1# | Fair rinsability |
| 2# | Fair rinsability |
| 3# | Precipitates were observed during the standing |
| 4# | Good rinsability |

1#, lauroyl sarcosinate salt 6% + benzoic acid 0.4% + fragrance 0.25% + balance of water (TO 100)
2#, lauroyl alanine 6% + arginine 3.84% + benzoic acid 0.2% + alkyl polyglycoside 1% + cocamidopropyl betaine 4.5% + glycerol 5% + fragrance 0.25% + balance of water (TO 100)
3#, sodium cocoyl glutamate 6% + benzoic acid 0.4% + fragrance 0.25% + balance of water (TO 100)
4#, lauroyl alanine 6% + arginine 3.84% + benzoic acid 0.2% + fragrance 0.25% + balance of water (TO 100)

Test II

An aqueous solution containing 6% of AES was prepared as cleaning liquid A, and an aqueous solution containing 6% of lauroyl alanine (neutralized by equimolar arginine) was prepared as liquid B. Cloths were washed to simulate the laundry process, and the rinsings were collected. The results are shown in FIG. 4. For the AES-containing cleaning liquid, a large quantity of foam was still observed after the first rinse with agitation on a shaker (A1 in FIG. 4), and substantially no foam was observed after the second rinse with agitation on a shaker (A2 in FIG. 4). For the lauroyl alanine (LA)-containing cleaning liquid, substantially no foam was observed after the first rinse with agitation on a shaker (B1 in FIG. 4).

**Example 8** <u>Wash feel and irritation</u>

5 assessors were requested for the assessment. Wash feel (direct application of the test samples on the arms/faces, followed by dry cleaning/no cleaning): non-stickiness 0, mild stickiness 1, moderate stickiness 2, severe stickiness 3.
5 assessors were requested for the assessment. Irritation (direct application of the test samples to the arms/faces, dry cleaning/no cleaning): mildness with no irritation 0, mild irritation 1, moderate irritation 2, severe irritation 3.

Table 7

|  | Wash feel (0-3) (average of scores from 5 assessors) | Irritation (0-3) (average of scores from 5 assessors) |
|---|---|---|
| 1# | 2 | 2 |
| 2# | 0.2 | 0.4 |
| 3# | 2.2 | 2 |
| 4# | 0.4 | 0.4 |
| 5# | 2 | 1 |
| 6# | 1.8 | 2 |
| 7# | Precipitates were observed during the standing | Precipitates were observed during the standing |
| 8# | 0.2 | 0 |

1#, lauroyl sarcosinate salt 6% + benzoic acid 0.4% + fragrance 0.25% + balance of water (TO 100)
2#, lauroyl alanine 6% + NaOH 0.87% + benzoic acid 0.4% + fragrance 0.25% + balance of water (TO 100)
3#, lauroyl alanine 6% + arginine 3.84% + benzoic acid 0.2% + alkyl polyglycoside 1% + cocamidopropyl betaine 4.5% + glycerol 5% + fragrance 0.25% + balance of water (TO 100)
4#, lauroyl alanine 6% + arginine 3.84% + benzoic acid 0.2% + alkyl polyglycoside 2% + fragrance 0.25% + balance of water (TO 100)
5#, lauroyl alanine 6% + arginine 3.84% + benzoic acid 0.2% + cocamidopropyl betaine 6% + fragrance 0.25% + balance of water (TO 100)
6#, lauroyl alanine 6% + arginine 3.84% + benzoic acid 0.2% + glycerol 10% + fragrance 0.25% + balance of water (TO 100)
7#, sodium cocoyl glutamate 6% + benzoic acid 0.4% + fragrance 0.25% + balance of water (TO 100)
8#, lauroyl alanine 6% + arginine 3.84% + benzoic acid 0.2% + fragrance 0.25% + balance of water (TO 100)

**Example 9** Oil control

1 Objective

The secretion of lipids in human skin is greatly influenced by androgens. Androgens stimulate the sebaceous glands to secrete lipids, while the $5\alpha$-reductase is a key enzyme for androgen metabolism. At present, the majority of available oil control products use ingredients that can effectively inhibit $5\alpha$-reductase to improve oil secretion and achieve the oil control effect.

2 Test item: inhibition rate of $5\alpha$-reductase.

3 Test materials

3.1 Main reagents

1) $5\alpha$-reductase: extracted from rat prostate tissue, preserved at -80 °C.
2) Sample preparation (TA): lauroyl alanine 6% + arginine 3.84% + benzoic acid 0.2% + fragrance 0.25% + balance of water (TO 100). The reaction concentration in the experimental system was 30% (V/V).
3) Blank control (BC): phosphate-buffered saline (PBS).

3.2 Main instrument: high-performance liquid chromatograph (HPLC).

4 Procedures

4.1 Preparation of $5\alpha$-reductase: extracted from male rat prostates. The sample was homogenized in a glass homogenizer with pre-cooled buffer at 0 °C in a ratio of 1:5 and centrifuged at 3000 g for 10 min. The supernatant was centrifuged at 10,000 g for 30 min and aliquoted into EP tubes at 1 mL/tube to give an enzyme extract, which was preserved in a freezer at -80 °C.

4.2 Determination of 5$\alpha$-reductase activity: A 2 mL reaction system contained 0.25 mL of phosphate buffer, 0.70 mL of the enzyme extract, 0.1 mL of a testosterone solution (0.5 mg/mL), 0.35 mL of a NADPH solution (1 mg/mL) and 0.6 mL of (10% ethanol for the complete reaction group and the sample solution for the sample group). The enzyme extract was replaced with a phosphate buffer in the control tube, and 2.0 mL of dichloromethane was added to terminate the reaction. The reaction in the sample tube was conducted at 37 °C for 30 min. After the reaction was completed, 2.0 mL of dichloromethane was added to stop the reaction. The mixture was shaken for 1 min and centrifuged at 5000 r/min for 10 min. The upper aqueous phase was discarded. About 1.0 mL of the organic phase was taken and concentrated by evaporation, and the residue was dissolved in 1.0 mL of methanol. The peak area of testosterone (T) was determined by high-performance liquid chromatography. 3 replicate tubes were set for each reaction. Calculation of inhibition rate:

$$\text{Inhibition rate \%} = \frac{T_{\text{sample tube}} - T_{\text{reaction tube}}}{T_{\text{blank tube}} - T_{\text{reaction tube}}} \times 100\%$$

5 Results

The results of 5$\alpha$-reductase activity inhibition rate are shown in Table 8.

Table 8. Summary of 5$\alpha$-reductase inhibition rate results

| Group | Concentration of system (%, V/V) | 5$\alpha$-reductase inhibition rate (%) | SD | p-value |
|---|---|---|---|---|
| Blank control (BC) | / | / | / | / |
| Negative control (NC) | / | 0.00 | 0.50 | / |
| Sample | 30 | 69.77 | 0.43 | 0.002** |

Note: When the statistical analysis was performed by using the t-test, the sample group was compared with the complete reaction group, and the significance was indicated by *.
* denotes $p < 0.05$, and ** denotes $p < 0.01$.

At a system concentration of 30% (V/V), the 5$\alpha$-reductase inhibition rate of the sample was 69.77%, with a statistical difference ($p < 0.01$) as compared to the NC group.

**Example** 10 Patch test for no-rinse cleaning or dry-cleanable cleaning liquid

Table 9

| | Ingredient | Amount (W%) |
|---|---|---|
| A | Water | 89.71 |
| B | Lauroyl alanine | 6 |
| C | Arginine | 3.84 |
| D | Benzoic acid | 0.2 |
| E | Fragrance | 0.25 |

The ingredients A, B, and C were dissolved by heating at 80-85 °C, and the mixture was stirred until complete dissolution and clarification. Then the mixture was cooled to 45 °C or less, and D and E were added. The mixture was stirred until complete dissolution and uniformity. A sample was taken and filtered, and the filtrate was discharged after being qualified.

A human skin patch test was conducted on the cleaning liquids described above.

1. Basis: SOP502-01CL - Single Human Skin Patch Test

2. Materials and procedures

2.1 Test substances: without treatment.
2.2 Negative control: The Grade III water in GB/T 6682 was used.
2.3 Subjects: A total of 30 subjects, 4 males and 26 females, aged 22 to 54 years.
2.4 Procedures: A qualified patch test device was selected. According to the sealing patch test procedures, about 0.02 mL of the test substance was applied to a patch tester, and the patch tester was applied to the upper back of the subject with a hypoallergenic tape. After 48 h, the test substance was removed. The skin reaction was observed 30 min after the removal. The results were recorded according to the skin reaction grading criteria in the Technical Specification for Cosmetic Safety (2015 edition).

3. Inclusion criteria

3.1 Volunteers aged 18-60 years who met the requirements of the test were selected as the subjects.

4. Results

Table 10

| Group | Number of subjects | Time of observation | Number of subjects with different skin reaction grades in patch test | | | | |
|---|---|---|---|---|---|---|---|
| | | | 0 | 1 | 2 | 3 | 4 |
| Test substance | 30 | 30min | 30 | 0 | 0 | 0 | 0 |
| Control | 30 | 30min | 30 | 0 | 0 | 0 | 0 |
| Note: The skin reactions of the 30 subjects receiving the single 48-hour patch test are shown in Table 10. | | | | | | | |

The results of the 48-hour irritation patch test show that in a single 48-hour patch test, adverse skin reactions occurred in 0 of the 30 subjects. This suggests that the cleaning liquid of the present disclosure does not require wash with water, and no adverse skin reaction occurs even if the cleaning liquid resides on the skin for 48 h.

Table 11. Skin reactions in a single human skin patch test in 30 subjects

| No. | Name (acronym) | Sex | Age | Negative control 30min | Test substance 30min |
|---|---|---|---|---|---|
| 1 | ZSS | Female | 25 | 0 | 0 |
| 2 | TYS | Female | 31 | 0 | 0 |
| 3 | XMH | Female | 37 | 0 | 0 |
| 4 | CXQ | Female | 32 | 0 | 0 |
| 5 | LM | Female | 24 | 0 | 0 |
| 6 | WJH | Female | 52 | 0 | 0 |
| 7 | JJH | Female | 54 | 0 | 0 |
| 8 | TSG | Female | 38 | 0 | 0 |
| 9 | SK | Male | 26 | 0 | 0 |
| 10 | FCG | Male | 24 | 0 | 0 |
| 11 | FCJ | Male | 24 | 0 | 0 |
| 12 | ZYX | Male | 34 | 0 | 0 |
| 13 | LJW | Female | 22 | 0 | 0 |
| 14 | LHH | Female | 29 | 0 | 0 |
| 15 | FJJ | Female | 22 | 0 | 0 |

(continued)

| No. | Name (acronym) | Sex | Age | Negative control 30min | Test substance 30min |
|---|---|---|---|---|---|
| 16 | LQL | Female | 47 | 0 | 0 |
| 17 | SYJ | Female | 24 | 0 | 0 |
| 18 | LWK | Female | 37 | 0 | 0 |
| 19 | HXZ | Female | 36 | 0 | 0 |
| 20 | ZJJ | Female | 35 | 0 | 0 |
| 21 | WF | Female | 22 | 0 | 0 |
| 22 | TZR | Female | 27 | 0 | 0 |
| 23 | LCX | Female | 33 | 0 | 0 |
| 24 | YQ | Female | 27 | 0 | 0 |
| 25 | DJ | Female | 30 | 0 | 0 |
| 26 | LHD | Female | 39 | 0 | 0 |
| 27 | CHL | Female | 34 | 0 | 0 |
| 28 | ZM | Female | 41 | 0 | 0 |
| 29 | JJF | Female | 25 | 0 | 0 |
| 30 | PHD | Female | 40 | 0 | 0 |

Further, an ultimate test was conducted by repeating the operations in section 2.4 above for a total of 3 times (i.e., 48 h × 3) as per SOP502-01CL - Multiple Human Skin Patch Tests. The results are shown in Table 12. Test substance 1 was obtained as per the formulas and preparation method in Table 9; test substance 2 was obtained as per the formulas and preparation method in Table 9, except that the preservative benzoic acid was replaced by sodium benzoate.

Table 12

| Group | Number of subjects | Cumulative number of patches | Number of subjects with different skin reaction grades in patch test | | | | |
|---|---|---|---|---|---|---|---|
| | | | 0 | 1 | 2 | 3 | 4 |
| Test substance 1 | 30 | I | 30 | 0 | 0 | 0 | 0 |
| | | III | 25 | 5 | 0 | 0 | 0 |
| Test substance 2 | 30 | I | 30 | 0 | 0 | 0 | 0 |
| | | III | 21 | 9 | 0 | 0 | 0 |
| Control | 30 | I | 30 | 0 | 0 | 0 | 0 |
| | | III | 30 | 0 | 0 | 0 | 0 |

The 48 h × 3 irritation patch test results show that after 3 cycles of patch tests, the number of people with skin reactions for test substance 2 reached 9 due to the replacement of the preservative with sodium benzoate, with a nearly 2-fold difference from that of test substance 1 using benzoic acid (5 people with skin reactions), indicating that the use of benzoic acid as a preservative significantly reduced the skin irritation.

**Example 11** Dry-cleanable shampoo (simplified formula)

Table 13. Formula of dry-cleanable shampoo

|   | Ingredient | Amount (W%) |
|---|---|---|
| A | Water | 89 |
| B | Lauroyl alanine | 6.6 |
| C | Arginine | 4.2 |
| D | Benzoic acid | 0.2 |

The ingredients A, B, and C were dissolved by heating at 80-85 °C and then cooled to 45 °C or less, before D was added.

Table 14. Formula of a commercially available shampoo

|   | Ingredient | Amount (W%) |
|---|---|---|
| 1 | Water | To 100 |
| 2 | Lauroyl alanine | 4 |
| 3 | Arginine | 2.6 |
| 4 | Polyquaternium-10 | 0.7 |
| 5 | Disodium EDTA | 0.1 |
| 6 | Cocamide methyl, MEA | 5 |
| 7 | TEA cocoylglutamate (30%) | 12 |
| 8 | Glycerol | 5 |
| 9 | Sodium lauroyl sarcosinate (30%) | 12 |
| 10 | Sodium benzoate | 0.4 |
| 11 | Citric acid | 0.01 |
| 12 | Sodium citrate | 0.01 |
| 13 | Cocamidopropyl betaine (30%) | 3.5 |
| 14 | Lauramidopropyl betaine (30%) | 3.5 |
| 15 | Phenoxyethanol | 0.8 |
| 16 | (Daily) fragrance | 0.2 |
| 17 | Polysorbate-20 | 0.5 |

Table 15. Performance assessment

|   | Shampoo of Example 11 | Commercially available shampoo |
|---|---|---|
| Combability | Good | Partially sticky |
| Refreshing after wash | Good | Thick and heavy |
| Fluffy after wash | Good | Hardened hair after dry cleaning |

The use of the dry-cleanable shampoo of this example provided a very refreshing feel after washing, and the hair was fluffy and combable (see a1-a3 in FIG. 5). Commercially available conventional shampoos, even when lauroyl alanine-arginine was also used, cannot be used as dry-cleanable shampoos if they are not used as the primary surfactant and the amount of other co-surfactants added is not controlled. During the dry cleaning process, the hair tended to stick together, like using mousse for styling, which makes the user feel heavy and sticky (see b1-b2 in FIG. 5). If a little water was added along with kneading, a large amount of foam also appeared (see b3 in FIG. 5).

**Example 12** Facial cleansing liquid (basic formula, simplified formula)

Table 16

| | Ingredient | Amount (W%) |
|---|---|---|
| A | Water | 89 |
| B | Lauroyl alanine | 6.6 |
| C | Arginine | 4.2 |
| D | Benzoic acid | 0.2 |

The ingredients A, B, and C were dissolved by heating at 80-85 °C, and the mixture was stirred until complete dissolution and clarification. Then the mixture was cooled to 45 °C or less, and D was added. The mixture was stirred until complete dissolution and uniformity. A sample was taken and filtered, and the filtrate was discharged after being qualified.
The basic formula of the facial cleansing liquid produced a rich and dense foam (see FIG. 6), which was not tight after wash, and can effectively remove dirt on the face while being mild and non-irritating.

**Example 13** Makeup removal cleaning liquid (basic formula, simplified formula)

Table 17

| | Ingredient | Amount (W%) |
|---|---|---|
| A | Water | 89 |
| B | Lauroyl alanine | 6.6 |
| C | Arginine | 4.2 |
| D | Benzoic acid | 0.2 |

The ingredients A, B, and C were dissolved by heating at 80-85 °C, and the mixture was stirred until complete dissolution and clarification. Then the mixture was cooled to 45 °C or less, and D was added. The mixture was stirred until complete dissolution and uniformity. A sample was taken and filtered, and the filtrate was discharged after being qualified.
The basic formula of the makeup removal cleaning liquid can effectively remove conventional colored cosmetics such as lipstick, brow powder, blusher, eye shadow, etc. (see FIG. 7).

**Example 14** Shaving cleaning liquid (basic formula, simplified formula)

Table 18

| | Ingredient | Amount (W%) |
|---|---|---|
| A | Water | 89 |
| B | Lauroyl alanine | 6.6 |
| C | Arginine | 4.2 |
| D | Benzoic acid | 0.2 |

The ingredients A, B, and C were dissolved by heating at 80-85 °C, and the mixture was stirred until complete dissolution and clarification. Then the mixture was cooled to 45 °C or less, and D was added. The mixture was stirred until complete dissolution and uniformity. A sample was taken and filtered, and the filtrate was discharged after being qualified.
Components of a commercially available shaving cleaning liquid: water, cetearyl alcohol, glyceryl stearate, cetrimonium chloride, fragrance, propylene glycol, benzyl alcohol, and methylisothiazolinone.

Two razors of the same brand and the same model were separately used in combination with the shaving cleaning liquid of Example 14 and the commercially available shaving cleaning liquid for shaving. After the shaving was completed, the razors were left to stand in the same conditions for 48 h. As shown in FIG. 8, the commercially available shaving cleaning liquid tends to adhere to the razor very easily, and is extremely difficult to clean if one forgets to process it promptly. However, the shaving cleaning liquid according to Example 14 does not easily adhere to the razor even if the washing is forgotten after use.

Moreover, those skilled in the art will appreciate that although some embodiments described herein include some features included in other embodiments, the combinations of features in different embodiments shall fall within the scope of the present disclosure and form different embodiments. For example, in the Claims, any of the claimed embodiments may be used in arbitrary combinations.

**Claims**

1. A cleaning composition, comprising: (a) a primary surfactant, (b) a co-surfactant, and (c) water, wherein the percentage by weight of the primary surfactant in all surfactants is greater than 50 wt%, preferably greater than 65 wt%, and more preferably greater than 80 wt%;

   the primary surfactant consists of a N-long-chain acyl amino acid and a basic amino acid; preferably, the percentage by weight of the N-long-chain acyl amino acid in the composition is 1-12 wt%, and the percentage by weight of the basic amino acid in the composition is 0.5-8 wt%;
   or, the primary surfactant consists of a N-long-chain acyl amino acid, a N-long-chain acyl amino acid dipeptide, and a basic amino acid; preferably, the sum of the percentages by weight of the N-long-chain acyl amino acid and the N-long-chain acyl amino acid dipeptide in the composition is 1-12 wt%, and the percentage by weight of the basic amino acid in the composition is 0.5-8 wt%;
   the percentage by weight of the co-surfactant in the cleaning composition is 0-10 wt%, preferably 0-5 wt%, and more preferably 0-1 wt%.

2. The cleaning composition according to claim 1, wherein the cleaning composition is substantially free of a thickening agent, preferably completely free of a thickening agent;

   and/or substantially free of a sulfate, preferably completely free of a sulfate;
   and/or substantially free of an ethoxylate, preferably completely free of an ethoxylate;
   and/or substantially free of a fatty acid salt, preferably completely free of a fatty acid salt;
   and/or substantially free of a polyol humectant, preferably completely free of a polyol humectant.

3. The cleaning composition according to claim 1, wherein the N-long-chain acyl group in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl;
   and/or, the amino acid in the N-long-chain acyl amino acid is derived from one or more of alanine, glycine, glutamic acid, sarcosine, arginine, lysine, or (methyl) taurine, preferably alanine, and most preferably L-alanine.

4. The cleaning composition according to claim 1, wherein the basic amino acid is selected from one or more of arginine, lysine, citrulline, ornithine, creatine, histidine, diaminobutyric acid, and diaminopropionic acid, preferably arginine and/or lysine, and most preferably arginine.

5. The cleaning composition according to claim 1, further comprising: (d) a preservative, wherein the preservative is benzoic acid.

6. The cleaning composition according to claim 1, wherein the pH of the composition is 5-7.5, preferably 5.5-6.9, and more preferably 6-6.5;
   and/or, the composition possesses a low viscosity; the low viscosity refers to a viscosity at 25 °C of less than 150 mPa·s, preferably less than 120 mPa·s, and more preferably less than 100 mPa·s.

7. The cleaning composition according to claim 1, wherein for the detergency of the composition on a specified stained fabric as measured by the test method in QB/T1224-2012 Liquid Detergent for Fabric, the JB-01 is 0.8 or higher, the JB-02 is 0.8 or higher, and the JB-03 is 0.4 or higher and 0.8 or lower.

8. The cleaning composition according to claim 1, wherein the composition has better foaming performance in hard water than in soft water; and/or, the foam volumes at 30 s, 3 min, and 5 min in hard water are greater than 400 mL, preferably greater than 450 mL, and more preferably greater than 500 mL; standard for the aforementioned test: GB/T7462-1994 "Surfactant: Measurement of Foaming Performance-Modified Ross-Miles Method", instrument: 2152 modified Ross-Miles foaming tester.

9. The cleaning composition according to any one of claims 1-8, wherein the cleaning composition is a body wash, a shampoo, a facial mousse, a makeup remover, a mouth wash, a shaving product, a hand sanitizer, or a multi-functional cleaning product.

10. Use of the cleaning composition according to any one of claims 1-8 in cleaning for the elderly, the sick, or the disabled population, or in cleaning for infants or children.

11. Use of the cleaning composition according to any one of claims 1-8 in treating or cleaning dry, aged, or damaged skin, or in intimate care.

12. A no-rinse or dry-cleanable cleaning liquid, comprising the composition according to any one of claims 1-8.

13. A pet cleanser, comprising the composition according to any one of claims 1-8.

14. A shampoo or a two-in-one cleanser for shampoo and body wash for infants or the elderly, comprising the composition according to any one of claims 1-8.

15. A method for reducing the skin irritation or eye irritation in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;
the method comprises: selecting a primary surfactant consisting of a N-long-chain acyl amino acid and a basic amino acid; or selecting a primary surfactant consisting of a N-long-chain acyl amino acid, a N-long-chain acyl amino acid dipeptide, and a basic amino acid.

16. The method according to claim 15, wherein the cleaning composition is substantially free of a thickening agent, preferably completely free of a thickening agent;

and/or substantially free of a sulfate, preferably completely free of a sulfate;
and/or substantially free of an ethoxylate, preferably completely free of an ethoxylate;
and/or substantially free of a fatty acid salt, preferably completely free of a fatty acid salt;
and/or substantially free of a polyol humectant, preferably completely free of a polyol humectant.

17. The method according to claim 15, wherein a preservative in the cleaning composition is benzoic acid.

18. The method according to claim 15, wherein the N-long-chain acyl amino acid is lauroyl alanine, the basic amino acid is arginine, and the N-long-chain acyl amino acid dipeptide is lauroyl alanyl alanine.

19. A method for improving the easy-to-rinse or water-saving performance in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;
the method comprises: selecting a primary surfactant consisting of a N-long-chain acyl amino acid and a basic amino acid; or selecting a primary surfactant consisting of a N-long-chain acyl amino acid, a N-long-chain acyl amino acid dipeptide, and a basic amino acid; or selecting a primary surfactant consisting of a N-long-chain acyl amino acid and an inorganic base.

20. The method according to claim 19, wherein the cleaning composition is substantially free of a thickening agent, preferably completely free of a thickening agent;

and/or substantially free of a sulfate, preferably completely free of a sulfate;
and/or substantially free of an ethoxylate, preferably completely free of an ethoxylate;

and/or substantially free of a fatty acid salt, preferably completely free of a fatty acid salt;
and/or substantially free of a polyol humectant, preferably completely free of a polyol humectant.

21. The method according to claim 19, wherein the N-long-chain acyl amino acid is lauroyl alanine, the basic amino acid is arginine, and the N-long-chain acyl amino acid dipeptide is lauroyl alanyl alanine.

22. A method for improving the deodorization performance in a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;

the method comprises: selecting a primary surfactant consisting of a N-long-chain acyl amino acid and a basic amino acid, wherein the basic amino acid is present in an amount partially neutralizing the N-long-chain acyl amino acid, preferably with the degree of neutralization being 85% or greater and less than 100%, more preferably 88% or greater and 96% or less;
or, selecting a primary surfactant consisting of a N-long-chain acyl amino acid, a N-long-chain acyl amino acid dipeptide, and a basic amino acid, wherein the basic amino acid is present in an amount partially neutralizing the N-long-chain acyl amino acid and the N-long-chain acyl amino acid dipeptide, preferably with the degree of neutralization being 85% or greater and less than 100%, more preferably 88% or greater and 96% or less;
or, selecting a primary surfactant consisting of a N-long-chain acyl amino acid and an inorganic base, wherein the inorganic base is present in an amount partially neutralizing the N-long-chain acyl amino acid, preferably with the degree of neutralization being 85% or greater and less than 100%, more preferably 88% or greater and 96% or less.

23. The method according to claim 22, wherein the N-long-chain acyl amino acid is lauroyl alanine, the basic amino acid is arginine, and the N-long-chain acyl amino acid dipeptide is lauroyl alanyl alanine.

24. A method for imparting the oil control performance to a cleaning composition, wherein the cleaning composition comprises: (a) a primary surfactant, (b) a co-surfactant, and (c) water; the percentage by weight of the primary surfactant in all surfactants is greater than 50 wt%, preferably greater than 65 wt%, and more preferably greater than 80 wt%;
the method comprises: selecting a primary surfactant consisting of a N-long-chain acyl amino acid and a basic amino acid; or selecting a primary surfactant consisting of a N-long-chain acyl amino acid, a N-long-chain acyl amino acid dipeptide, and a basic amino acid; or selecting a primary surfactant consisting of a N-long-chain acyl amino acid and an inorganic base.

25. The method according to claim 24, wherein the N-long-chain acyl amino acid is lauroyl alanine, the basic amino acid is arginine, and the N-long-chain acyl amino acid dipeptide is lauroyl alanyl alanine.

FIG. 1

FIG. 2

FIG. 3

FIG. 4

FIG. 5

FIG. 6

FIG. 7

FIG. 8

## INTERNATIONAL SEARCH REPORT

| International application No. |
|---|
| **PCT/CN2024/102882** |

### A. CLASSIFICATION OF SUBJECT MATTER

A61K 8/44(2006.01)i;  A61K 8/64(2006.01)i;  A61Q 19/10(2006.01)i;  A61Q 5/02(2006.01)i;  A61Q 19/00(2006.01)i;  C11D 7/34(2006.01)i;  C11D 7/32(2006.01)i

According to International Patent Classification (IPC) or to both national classification and IPC

### B. FIELDS SEARCHED

Minimum documentation searched (classification system followed by classification symbols)

IPC:  A61K,  A61Q,  C11D

Documentation searched other than minimum documentation to the extent that such documents are included in the fields searched

Electronic data base consulted during the international search (name of data base and, where practicable, search terms used)

CNTXT, CNABS, DWPI, VEN, ENTXT, CNKI, 万方, WANFANG, ISI Web of Knowledge: 清洁, 洗涤, 表面活性剂, N-长链酰基氨基酸, N-长链酰基氨基酸二肽, 碱性氨基酸, 无机碱, 月桂酰丙氨酸, 月桂酰丙氨酰丙氨酸, 精氨酸, 苯甲酸, 刺激, 冲洗, 节水, 除味, 控油, cleaning, washing, N-long-chain acyl amino acid, N-long-chain acyl amino acid dipeptide, basic amino acids, inorganic base, lauroyl alanine, Lauroyl alanyl alanine, arginine, benzoic acid, stimulation, rinsing, water conservation, odor removal, oil control

### C. DOCUMENTS CONSIDERED TO BE RELEVANT

| Category* | Citation of document, with indication, where appropriate, of the relevant passages | Relevant to claim No. |
|---|---|---|
| PX | WO 2024078286 A1 (SUZHOU OULIT BIOPHARM CO., LTD.) 18 April 2024 (2024-04-18) <br> claims 1-29 | 1-25 |
| X | CN 115700272 A (SUZHOU OULIT BIOPHARM CO., LTD.) 07 February 2023 (2023-02-07) <br> description, paragraphs 314-315, 317, 322, 630, 650, and 655-656, embodiment 6, and table 11b of application example 9 | 1-25 |
| A | WO 2019233377 A1 (SUZHOU OULIT BIOPHARM CO., LTD.) 12 December 2019 (2019-12-12) <br> claims 1-49 | 1-25 |
| A | US 2022073683 A1 (SUZHOU OULIT BIOPHARM CO., LTD.) 10 March 2022 (2022-03-10) <br> claims 1-50, and abstract | 1-25 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 September 2024** | **08 October 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| PCT/CN2024/102882 |

## C. DOCUMENTS CONSIDERED TO BE RELEVANT

| Category* | Citation of document, with indication, where appropriate, of the relevant passages | Relevant to claim No. |
|---|---|---|
| A | US 2006239952 A1 (AJINOMOTO CO., INC.) 26 October 2006 (2006-10-26) claims 1-22, and description, embodiments 1-6 | 1-25 |
| A | CN 106619162 A (JIUJIANG TINCI MATERIALS TECHNOLOGY CO., LTD.) 10 May 2017 (2017-05-10) claims 1-8 | 1-25 |
| A | CN 1735682 A (PIF-CO., LTD.) 15 February 2006 (2006-02-15) claims 1-14, and description, embodiments 1-7 | 1-25 |

Form PCT/ISA/210 (second sheet) (July 2022)

## INTERNATIONAL SEARCH REPORT
### Information on patent family members

| International application No. |
| --- |
| **PCT/CN2024/102882** |

| Patent document cited in search report | | | Publication date (day/month/year) | Patent family member(s) | | | Publication date (day/month/year) |
| --- | --- | --- | --- | --- | --- | --- | --- |
| WO | 2024078286 | A1 | 18 April 2024 | None | | | |
| CN | 115700272 | A | 07 February 2023 | BR | 112024001179 | A2 | 30 April 2024 |
| | | | | WO | 2023001267 | A1 | 26 January 2023 |
| | | | | CA | 3225766 | A1 | 26 January 2023 |
| | | | | JP | 2024527160 | A | 19 July 2024 |
| | | | | MX | 2024001064 | A | 27 February 2024 |
| | | | | KR | 20240050341 | A | 18 April 2024 |
| | | | | EP | 4375270 | A1 | 29 May 2024 |
| | | | | AU | 2022313377 | A1 | 29 February 2024 |
| WO | 2019233377 | A1 | 12 December 2019 | JP | 2021527118 | A | 11 October 2021 |
| | | | | JP | 7391397 | B2 | 05 December 2023 |
| | | | | EP | 3805249 | A1 | 14 April 2021 |
| | | | | EP | 3805249 | A4 | 27 April 2022 |
| | | | | US | 2021236405 | A1 | 05 August 2021 |
| | | | | US | 11540993 | B2 | 03 January 2023 |
| US | 2022073683 | A1 | 10 March 2022 | US | 11566110 | B2 | 31 January 2023 |
| | | | | KR | 20210018331 | A | 17 February 2021 |
| | | | | EP | 3805200 | A1 | 14 April 2021 |
| | | | | EP | 3805200 | A4 | 27 April 2022 |
| | | | | BR | 112020024723 | A2 | 23 March 2021 |
| | | | | AU | 2019280295 | A1 | 21 January 2021 |
| | | | | AU | 2019280295 | B2 | 19 January 2023 |
| | | | | JP | 2021527117 | A | 11 October 2021 |
| | | | | JP | 7502800 | B2 | 19 June 2024 |
| | | | | WO | 2019233375 | A1 | 12 December 2019 |
| | | | | MX | 2020013130 | A | 29 April 2021 |
| US | 2006239952 | A1 | 26 October 2006 | WO | 2005033255 | A1 | 14 April 2005 |
| | | | | EP | 1672055 | A1 | 21 June 2006 |
| | | | | EP | 1672055 | A4 | 13 December 2006 |
| | | | | EP | 1672055 | B1 | 01 October 2008 |
| | | | | DE | 602004016873 | D1 | 13 November 2008 |
| | | | | ES | 2313068 | T3 | 01 March 2009 |
| | | | | JPWO | 2005033255 | A1 | 14 December 2006 |
| | | | | JP | 4771134 | B2 | 14 September 2011 |
| | | | | BRPI | 0414953 | A | 07 November 2006 |
| | | | | BRPI | 0414953 | B1 | 25 October 2016 |
| CN | 106619162 | A | 10 May 2017 | None | | | |
| CN | 1735682 | A | 15 February 2006 | JPWO | 2004061060 | A1 | 11 May 2006 |
| | | | | JP | 4644493 | B2 | 02 March 2011 |
| | | | | ES | 2327833 | T3 | 04 November 2009 |
| | | | | EP | 1586625 | A1 | 19 October 2005 |
| | | | | EP | 1586625 | A4 | 07 June 2006 |
| | | | | EP | 1586625 | B1 | 03 June 2009 |
| | | | | DE | 60327892 | D1 | 16 July 2009 |
| | | | | WO | 2004061060 | A1 | 22 July 2004 |
| | | | | US | 2006062751 | A1 | 23 March 2006 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3582748 A1
- GB 2598154 A
- EP 3957297 A1
- CN 105997546 B
- GB 74621994 T
- CN 1798821 A
- US 6703517 B2
- CN 102875409 B
- JP H0570418 A
- CN 202210867760
- CN 2022107270 W
- CN 100448968C